# EUROPÄISCHE PATENTSCHRIFT

(11) **EP 2 181 129 B1**
(45) Veröffentlichungstag und Bekanntmachung des Hinweises auf die Patenterteilung: **04.05.2011**
(21) Anmeldenummer: 08784725.7
(22) Anmeldetag: 11.07.2008
(51) Int. Cl.: C08F 220/38, C07C 323/54, C08F 218/08

(54) **VERNETZBARE MONOMERE UND POLYMERE UND DEREN VERWENDUNG**
CROSS-LINKABLE MONOMERS AND POLYMERS AND THE USE THEREOF
MONOMÈRES ET POLYMÈRES RÉTICULABLES, ET LEUR UTILISATION

(30) Priorität: 20.08.2007 DE 102007039312
(43) Veröffentlichungstag der Anmeldung: 05.05.2010
(73) Patentinhaber: Celanese Emulsions GmbH, 61476 Kronberg/Ts. (DE)
(72) Erfinder: JAKOB, Martin, 65779 Kelkheim (DE); NOGAI, Stefan, 69120 Heidelberg (DE)
(74) Vertreter: von Kreisler Selting Werner
(86) Internationale Anmeldenummer: PCT/EP2008/005686
(87) Internationale Veröffentlichungsnummer: WO 2009/024216

(56) Entgegenhaltungen:
- DE-A1- 2 058 773
- GB-A- 1 322 971

## Beschreibung

Die vorliegende Erfindung betrifft vernetzbare Monomere sowie davon abgeleitete Copolymere, insbesondere in der Form von wässrigen Polymerdispersionen. Verfilmungen aus diesen Copolymeren zeichnen sich durch eine gute Wasserresistenz aus und sind formaldehydfrei. Die Erfindung betrifft ferner die Verwendung dieser Monomeren und Copolymeren, beispielsweise zum Verkleben von Substraten aller Art.

Wäßrige Polymerdispersionen, darunter solche auf Basis von Polyvinylestern, wie homopolymerem Polyvinylacetat oder Copolymeren aus Vinylacetat und Ethylen, haben weite industrielle Verbreitung als Klebstoffe, Beschichtungs- oder Bindemittel gefunden. Diese Polymeren können nach Wasser- oder Lösemittelbelastung stark an Kohäsion verlieren, was durch Zusatz von einpolymerisierten Vernetzermonomeren oder extern zugesetzten Verbindungen wie selektiv ausgewählten Harzen auf Harnstoff-, Melamin-, Phenol- oder Glyoxalbasis verringert werden kann. Ein gängiges Vernetzermonomer ist dabei N-Methylolacrylamid (nachstehend "NMA"). Die N-Methylolqruppe des NMA (oder auch N-Methylolmethacrylamid) kann nachträglich selbstvernetzen und dadurch die Filmkohäsion verbessern, aber auch über Amidomethylierung insbesondere von Hydroxylgruppen eine kovalente Anbindung des Emulsionspolymeren an hydroxyfunktionelle Stabilisierungsmittel, wie an Celluloseether oder an Polyvinylalkohol oder an Substratoberflächen, wie an Textilien, Holz oder Papier gestatten. Die technischen Handelsformen dieses Produkts können jedoch bis zu 2 Gew.-% freien Formaldehyd enthalten, welcher in die Dispersionen eingetragen wird. Auch bei der Vernetzungreaktion selbst wird - abhängig von den gewählten Bedingungen - Formaldehyd freigesetzt. Mögliche Mechanismen dafür werden in der Literatur, beispielsweise von K. Hübner und F. Kollinsky, Angew. Makromol. Chem. 11, 125-134 (1970) beschrieben. Formaldehyd ist ein Gefahrstoff mit reizender und sensibilisierender Wirkung. Darüberhinaus wird ihm seit geraumer Zeit auch ein krebserregendes Potential zugeschrieben. In der Vergangenheit sind deshalb viele Versuche unternommen worden, funktionelle vernetzbare Monomere mit ähnlichem Potential, wie N-Methylolacrylamid oder N-Methylolmethacrylamid, zu entwickeln und in Polymerdispersionen als funktionelle Komponente zu verwenden.

Veretherte N-Methylolmonomere, wie N-Methoxymethyl(meth)acrylamid, N-Butoxymethyl(meth)acrylamid und N-Isobutoxymethyl(meth)acrylamid, sind seit längerem bekannt. Diese besitzen jedoch für viele Anwendungen nicht die geforderten Reaktivitäten, insbesondere nicht bei Anwendungen, wo die Vernetzung schon bei Raumtemperatur gefordert wird. Die Produkte stellen für viele Anwendungen demnach keinen Ersatz dar.

US-A-5,021,529 beschreibt Formaldehyd-freie Interpolymere, geeignet für die Produktion in impregnierten oder behandelten Papieren, Textil und Non-Wovens. Als Vernetzermonomere werden N-Ethylol(meth)acrylamid und -malimid, N-Propylol(meth)acrylamid, N-Butylol-(meth)acrylamid- und -malimid sowie N-Benzylol(meth)acrylamid vorgeschlagen. Zur Härtung, beispielsweise bei der Ausrüstung von textilen Geweben eignen sich Temperaturbereiche zwischen 120°C und 160 °C.

EP-A-514,654 beschreibt Formaldehyd-freie vernetzende Emulsionspolymere, abgeleitet von N-(2,2,-Dialkoxy-1-hydroxy)ethylacrylamid und Vinylestern. Die so erhaltenen Dispersionen eignen sich als Binder für Non-Wovens oder Fiberfill, sowie als Holzklebstoffe. Die Reaktivität dieses Systems entspricht der eines über N-Methylolacrylamid vernetzten Polymeren. Im Falle der Holzverleimung wird eine ausreichende Reaktivität nach thermischer Aktivierung beim Heißverkleben erreicht. Dieses wird in Beispiel 18 des Dokuments dargelegt.

Prinzipiell länger bekannt sind Aldehyd-funktionelle Monomere. Die EP-A-003,516 schlägt beispielsweise (Meth)acryloxyalkylpropanale als Vernetzer vor. Diese werden leicht durch Veresterung von β-Hydroxyalkylpropanalen erhalten. Diese carbonylfunktionellen Monomere können mit Polyhydrazinen unter Hydrazidbildung vernetzen. Aus der US-A-5,258,477 ist allerdings bekannt, daß die freie Aldehydgruppe solcher Monomerer während der Polymerisation zu Ketten-transfer neigt und die Polymeren auf diese Weise vorvernetzt. Dadurch steht ein Teil der reaktiven Gruppen für die End-Applikation nicht mehr zur Verfügung.

In der US-A-5,258,477 wird dieser technische Nachteil durch Maskierung der Aldehydmonomere als Acetal umgangen. Hier werden zahlreiche Strukturen auf Basis von mit 2,2-Dimethyl-3-hydroxypropanal veresterter Croton- Malein-, Fumar- oder Itaconsäure vorgeschlagen. Diese Ester werden mit mono- oder difunktionellen Alkoholen zu offenen oder cyclischen Acetalen umgesetzt. Emulsionspolymere mit diesen Acetalmonomeren eignen sich als Bindemittel für Non-Wovens.

Zur Verbesserung der Naßfestigkeit von Papier kommen in der WO-A-98/54,237 Acetalmonomere auf (Meth)acrylat oder (Meth)acrylamid-Basis zusammen mit selektiv ausgewählten kationischen Monomeren zum Einsatz. Beispielhaft genannt seien die Acetale N-(2,2-dimethoxyethyl)-N-methylacrylamid und 3,3-dimethoxy-1-methylethylacrylat.

Emulsionspolymere auf Vinylesterbasis, insbesondere Vinylacetat, sind üblicherweise schwach sauer eingestellt. In dieser Umgebung erleiden die Acetale eine langsame irreversible Hydrolyse unter Freisetzung der reaktiven Aldehyde. Sind in der Dispersion funktionelle Gruppen vorhanden, die mit den Aldehyden reagieren können, beispielsweise bei Verwendung von Polyvinylalkohol als Schutzkolloid, so führt dies zu einer unerwünschten Reduktion der Lagerstabilität. Die bei der Hydrolyse der Acetale freigesetzten Alkohole erhöhen den Gehalt an VOC (Volatile Organic Compounds), was bei einigen Applikationen unerwünscht ist.

Deswegen besteht am Markt nach wie vor ein Bedarf nach funktionellen Comonomeren, die sich zur Herstellung vernetzbarer Polymere eigenen, die insbesondere aus Emulsion ohne Freisetzung von Formaldehyd effektiv vernetzen, ohne die Nachteile der Systeme des Standes der Technik aufzuweisen.

Demnach liegt dieser Erfindung die Aufgabe zugrunde, Vernetzermonomere und davon abgeleitete Copolymere bereitzustellen, welche neben ausgezeichneter Lagerstabilität eine effektive Vernetzung gestatten und welche keine Formaldehydquellen darstellen.

Eine weitere Aufgabe der vorliegenden Erfindung besteht in der Bereitstellung von Vernetzermonomeren und davon abgeleiteten Copolymeren, welche bei Raumtemperatur vernetzen und welche in der Lage sind, mit Hydroxy-funktionellen Gruppen zu reagieren. Bei diesen Gruppen kann es sich beispielsweise um Gruppen von Bestandteilen einer Formulierung, beispielsweise eines Emulsionspolymerisats, wie Gruppen in Stabilisierungsmitteln handeln, oder es kann sich um Gruppen in Substraten handeln, beispielsweise solchen in cellulosischen Substraten, wie Textilien, Papier und Holz.

Noch eine weitere Aufgabe der vorliegenden Erfindung besteht in der Bereitstellung von Vernetzermonomeren und davon abgeleiteten Copolymeren, welche leicht zugänglich sind.

Diese Aufgaben werden durch die nachstehend beschriebenen Monomeren und Copolymeren gelöst.

Die Erfindung betrifft Copolymerisate abgeleitet von mindestens einem Monomer in Säure- oder Salzform, das ein Anion der Formel I und ein oder mehrere Kationen zur Herstellung der Elektroneutralität enthält, und mindestens einem weiteren damit radikalisch copolymerisierbaren Monomer 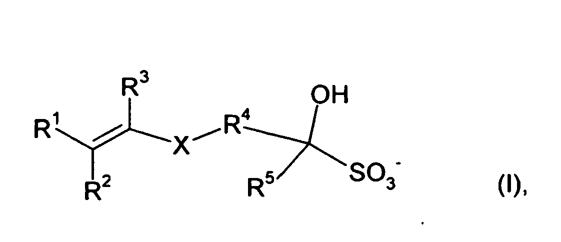 worin
R¹ und R² unabhängig voneinander Wasserstoff, Alkyl, Cycloalkyl, Aryl, Aralkyl, -COOR⁵, -COO⁻ Kat⁺ oder -CON(R⁶R⁷) bedeuten,
R⁶ und R⁷ unabhängig voneinander Wasserstoff, Alkyl oder Aryl sind,
Kat⁺ ein einwertiges Kation bedeutet, und
einer der Reste R¹ oder R² auch eine Gruppe -X-R⁴-CR⁵(OH)(SO₃) bedeuten kann,
wobei X, R⁴ und R⁵ eine der unten genannten Bedeutungen annehmen,
R³ Wasserstoff, Alkyl oder Aryl bedeutet,
X ausgewählt wird aus der Gruppe direkte C-C-Bindung, -O-, -CH₂-O-, -CH₂-NR⁸-, -COO- oder -CONR⁸-,
R⁸ = Wasserstoff, Alkyl oder Aryl bedeutet,
R⁴ Alkylen, Polyoxyalkylen, Cycloalkylen oder Arylen bedeutet, und
R⁵ Wasserstoff, Alkyl, Cycloalkyl oder Aryl bedeutet.

Die Erfindung betrifft außerdem Verbindungen in Säure- oder Salzform, die ein Anion der Formel (I) und ein oder mehrere Kationen zur Herstellung der Elektroneutralität enthalten.

Diese Verbindungen zeichnen sich durch eine hohe Stabilität aus und können als Festsubstanz isoliert werden. Die entsprechenden Vorläufersubstanzen, z.B. die Aldehyde, liegen häufig in flüssiger Form vor und sind teilweise chemisch labil. Können Gruppen oder Substituenten mehrfach in den Verbindungen der Formel (I) vorkommen, so können sie unabhängig voneinander die angegebenen Bedeutungen haben und können jeweils gleich oder verschieden sein.

Die Anionen und Kationen der Verbindungen der Formel (I) sind so zu wählen, dass eine elektrisch neutrale Verbindung entsteht.

Bei den Kationen zur Herstellung der Elektroneutralität handelt es sich in der Regel um ein- bis vierwertige Kationen, vorzugsweise um Wasserstoff, um Amonium oder um ein ein- bis vierwertige Metallionen.

Bedeuten irgendwelche Gruppen Alkyl, so können diese geradkettig oder verzweigt sein. Dies gilt auch, wenn sie in anderen Gruppen enthalten sind, zum Beispiel in Alkoxygruppen, Alkoxycarbonylgruppen oder in Aminogruppen, oder wenn sie substituiert sind. Alkylreste enthalten üblicherweise ein bis achtzehn Kohlenstoffatome, vorzugsweise ein bis zehn Kohlenstoffatome, insbesondere ein bis acht Kohlenstoffatome. Alkylreste können ihrerseits substituiert sein, beispielsweise mit Cycloalkyl-, Alkoxy- oder Arylresten und/oder mit Halogen. Bevorzugt sind unsubstituierte Alkylreste.

Beispiele für Alkylgruppen sind Methyl, Ethyl, n-Propyl, n-Butyl, n-Pentyl, n-Hexyl, n-Heptyl, n-Octyl, n-Nonyl, n-Decyl, n-Dodecyl, n-Lauryl, n-Hexadecyl, n-Octadeyl, Isopropyl, Isobutyl, Isopentyl, sec-Butyl, tert-Butyl, Neopentyl, 3,3-Dimethylbutyl oder 2-Ethylhexyl.

Bedeuten irgendwelche Gruppen Cycloalkyl, so handelt es sich dabei um gesättigte monocyclische oder polycyclische Kohlenwasserstoffreste. Bevorzugt sind monocyclische Cycloalkylreste enthaltend fünf bis acht Ring-Kohlenstoffatome, vorzugsweise fünf oder sechs Ring-Kohlenstoffatome. Cycloalkylreste können ihrerseits substituiert sein, beispielsweise mit Alkyl-, Alkoxy- oder Arylresten und/oder mit Halogen. Bevorzugt sind unsubstituierte Cycloalkylreste.

Beispiele für Cycloalkylreste sind Cyclopentyl, Cyclohexyl, Cycloheptyl und Cycloctyl, die zum Beispiel durch einen oder mehrere gleiche oder verschiedene (C₁-C₄)-Alkylreste, insbesondere durch Methyl, substituiert sein können.

Bedeuten irgendwelche Gruppen Aryl, so handelt es sich dabei um carbocyclische oder heterocyclische aromatische Reste, vorzugsweise Phenyl, Naphtyl oder Heteroaryl. Arylreste können unsubstituiert oder ein- oder mehrfach substituiert sein. Beispiele für Substituenten sind Alkyl-, Alkoxy-, Hydroxy-, Amino-, Carboxyl- und/oder Carbonsäureestergruppen sowie Halogen. Bevorzugt sind alkylsubstituierte oder insbesondere unsubstituierte Arylreste. Ganz besonders bevorzugt ist Phenyl.

Heterocyclische aromatische Reste stehen vorzugsweise für 5- bis 7-gliedrige ungesättigte Heterocyclen, die ein oder mehrerer Heteroatome aus der Reihe O, N, S aufweisen. Die von diesen Heterocyclen abgeleiteten Reste können über jedes Ringkohlenstoffatom gebunden sein.

Bedeuten irgendwelche Gruppen Aralkyl, so handelt es sich dabei um Arylreste, die über eine Alkylengruppe mit dem entsprechenden Rest verbunden sind. Ein bevorzugtes Beispiel für einen Aralkylrest ist Benzyl. Aralkylreste können unsubstituiert oder ein- oder mehrfach substituiert sein. Beispiele für Substituenten sind bei der Beschreibung der Arylreste aufgeführt. Bevorzugt sind unsubstituierte Aralkylreste.

Halogen bedeutet Fluor, Chlor, Brom oder lod, vorzugsweise Chlor.

Bedeuten irgendwelche Gruppen Alkylen, so können diese geradkettig oder verzweigt sein. Bei Alkylenresten handelt es sich um zweiwertige aliphatische Kohlenwasserstoffreste. Diese enthalten üblicherweise ein bis achtzehn Kohlenstoffatome, vorzugsweise ein bis sechs Kohlenstoffatome. Alkylenreste können ihrerseits substituiert sein, beispielsweise mit Cycloalkyl-, Alkoxy-, Hydroxy- oder Arylresten und/oder mit Halogen. Bevorzugt sind unsubstituierte Alkylenreste.

Beispiele für Alkylengruppen sind Reste der allgemeinen Formel (II)

-CₘH₂ₘ- (II),

worin m eine ganze Zahl von 1 bis 18, insbesondere von 1 bis 8, ganz besonders bevorzugt von 1 bis 6 bedeutet.
Ganz besonders bevorzugte Alkylenreste sind Rest der Formeln -CH₂-, -CH₂-CH₂-, -CH(CH₃)-CH₂-, -(CH₂)₃- und -(CH₂)₄-.

Bedeuten irgendwelche Gruppen Polyoxyalkylen, so können diese geradkettig oder verzweigt sein. Bei Polyoxyalkylenresten handelt es sich um zweiwertige aliphatische Polyetherreste. Die wiederkehrenden Kohlenwasserstoffeinheiten dieser Polyetherreste enthalten üblicherweise zwei bis sechs Kohlenstoffatome, vorzugsweise zwei bis vier Kohlenstoffatome. Polyoxyalkylenreste können ihrerseits substituiert sein, beispielsweise mit Cycloalkyl-, Alkoxy- oder Arylresten und/oder mit Halogen. Bevorzugt sind unsubstituierte Polyoxyalkylenreste.

Beispiele für Polyoxyalkylengruppen sind Reste der allgemeinen Formel (III)

(-CₒH₂ₒ-O)ₚ- (III),

worin o eine ganze Zahl von 2 bis 6, insbesondere von 2 bis 4, ganz besonders bevorzugt 2 oder 3 bedeutet und worin p eine ganze Zahl von 2 bis 30, insbesondere 2 bis 10 ist.

Ganz besonders bevorzugte Polyoxyalkylenreste sind unsubstituierte Polyoxyethylenreste.

Bedeuten irgendwelche Gruppen Cycloalkylen, so handelt es sich dabei um gesättigte monocyclische oder polycyclische zweiwertige Kohlenwasserstoffreste. Bevorzugt sind monocyclische Cycloalkylenreste enthaltend fünf bis acht Ring-Kohlenstoffatome, vorzugsweise fünf oder sechs Ring-Kohlenstoffatome. Cycloalkylenreste können ihrerseits substituiert sein, beispielsweise mit Alkyl-, Alkoxy-, Hydroxy- oder Arylresten und/oder mit Halogen. Bevorzugt sind unsubstituierte Cycloalkylenreste.

Beispiele für Cycloalkylenreste sind Cyclopentylen und Cyclohexylen.

Bedeuten irgendwelche Gruppen Arylen, so handelt es sich dabei um carbocyclische oder heterocyclische zweiwertige aromatische Reste, vorzugsweise um Phenylen, Naphthylen oder Heteroarylen. Arylenreste können unsubstituiert oder ein- oder mehrfach substituiert sein. Beispiele für Substituenten sind Alkyl-, Alkoxy-, Hydroxy-, Amino-, Carboxyl- und/oder Carbonsäureestergruppen sowie Halogen. Bevorzugt sind alkylsubstituierte oder insbesondere unsubstituierte Arylenreste. Ganz besonders bevorzugt ist Phenylen. Dabei kann es sich um ortho-, meta- oder para-Phenylen handeln.

Heterocyclische Arylenreste stehen vorzugsweise für 5- bis 7-gliedrige ungesättigte Heterocyclen, die ein oder mehrerer Heteroatome aus der Reihe O, N, S aufweisen. Die von diesen Heterocyclen abgeleiteten Reste können über zwei ihrer Ringkohlenstoffatome gebunden.

Bedeuten irgendwelche Reste einwertige Kationen, so kann es sich dabei um beliebige einfach geladene Kationen handeln. Beispiele für bevorzugte einwertige Kationen sind kationischer Wasserstoff (das Proton), das Ammoniumkation oder Kationen einwertiger Metalle, insbesondere der Alkalimetalle, wie Natrium oder Kalium. Bevorzugt sind das Ammoniumkation oder Kationen der Alkalimetalle.

Bedeuten irgendwelche Reste ein- bis vierwertige Kationen, so kann es sich dabei um beliebige einfach bis vierfach geladene Kationen handeln. Beispiele für einfach geladene Kationen sind im voranstehenden Abschnitt aufgezählt. Beispiele für zweifach geladene Kationen sind Kationen zweiwertiger Metalle, insbesondere der Metalle der Erdalkalimetalle, ganz besonders bevorzugt des Magnesiums, Calciums oder Strontiums. Beispiele für dreifach geladene Kationen sind Kationen dreiwertiger Metalle, insbesondere der Metalle der dritten Haupt- und Nebengruppe des Periodensystems, ganz besonders bevorzugt des Aluminiums. Beispiele für vierfach geladene Kationen sind Kationen vierwertiger Metalle, insbesondere der Metalle der vierten Haupt- und Nebengruppe des Periodensystems, ganz besonders bevorzugt des Zinns, Zirkons und des Titans.

Bevorzugt sind Verbindungen der Formel (I), worin R¹, R² und R³ unabhängig voneinander Wasserstoff, Alkyl, -COOR⁵ oder -COO- Kat⁺ bedeuten.

Besonders bevorzugt sind Verbindungen der Formel (I), worin R¹. R² und R³ unabhängig voneinander Wasserstoff oder Alkyl und einer der Reste R¹ oder R² -COOR⁵ oder -COO- Kat⁺ bedeuten kann.

Ganz bevorzugt sind Verbindungen der Formel (I), worin R¹, R² und R³ unabhängig voneinander Wasserstoff oder Methyl und einer der Reste R¹ oder R² -COOR^{5a} oder -COO- Kat⁺ bedeuten kann, wobei R^{5a} Wasserstoff oder C₁-C₆-Alkyl ist.

Ebenfalls bevorzugt sind Verbindungen der Formel (I), worin einer der Reste R¹ oder R² eine Gruppe -X-R⁴-CR⁵(OH)(SO₃⁻) bedeutet.

Bevorzugt sind Verbindungen der Formel (I), worin X ausgewählt wird aus der Gruppe -O-, -CH₂-O-, -CO-NR⁸- oder -COO-, ganz besonders bevorzugt -COO- ist.

Ebenfalls bevorzugt sind Verbindungen der Formel (I), worin R⁴ Alkylen oder Polyoxyalkylen bedeutet, besonders bevorzugt unsubstituiertes C₁-C₆-Alkylen, und ganz besonders bevorzugt unsubstituiertes C₁-C₄-Alkylen.

Ebenfalls bevorzugt sind Verbindungen der Formel (I), worin R⁵ Wasserstoff oder Alkyl bedeutet, ganz besonders bevorzugt Wasserstoff.

Ebenfalls bevorzugt sind Verbindungen der Formel (I), worin n = 1 ist und worin M Ammonium oder ein einwertiges Metallion ist.

Ganz besonders bevorzugte Verbindungen der Formel (I) leiten sich von Acrylsäure oder von Methacrylsäure ab. Dabei handelt es sich um Verbindungen in Säure- oder Salzform, die ein Anion der Formel (IVa) oder der Formel (IVb) und ein oder mehrere Kationen zur Herstellung der Elektroneutralität enthalten 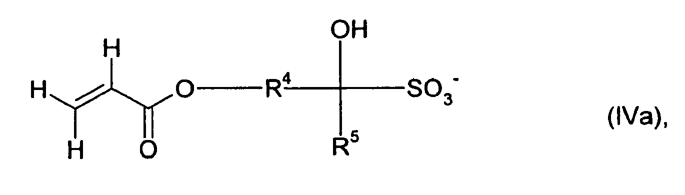 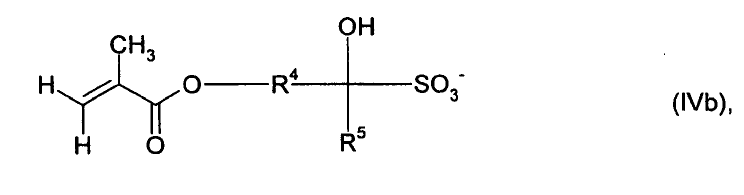 worin R⁴ und R⁵ die weiter oben definierte Bedeutung besitzen.

Grundsätzlich stellen die Verbindungen der Formel (I) formal eine Additionsverbindung eines Bisulfitsalzes an eine vinylisch ungesättigte Carbonylverbindung dar.

Zur Synthese der erfindungsgemäßen Vernetzermonomere der Formel (I) sind die entsprechenden Monomeren mit Carbonylgruppen, insbesondere mit Aldehydgruppen (R⁵=H), oder auch ihre Acetale und gegebenenfalls andere maskierte Formen als Vorstufe geeignet. Bei den Monomeren mit Carbonylgruppen handelt es sich um Verbindungen der Formel (V) 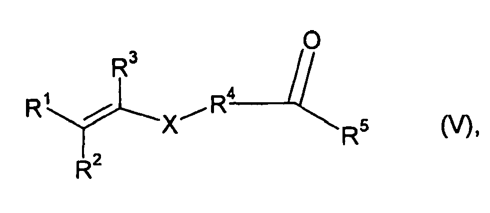 worin R¹, R², R³, R⁴, R⁵ und X die weitere oben definierte Bedeutung besitzen.

Bevorzugte Verbindungen der Formel (V) mit R⁴ = -C₃H₆- leiten sich von Acrylsäure oder von Methacrylsäure ab. Dabei handelt es sich um Verbindungen der Formel (V) mit R¹, R² und R³ gleich Wasserstoff sowie X = -CO-O- oder mit R¹ und R² gleich Wasserstoff und R³ gleich Methyl sowie X = -CO-O-.

Im Falle der Maskierung der Verbindungen der Formel (V) wird die Schutzgruppe vor oder während der Herstellung des Bisulfitaddukts unter geeigneten Reaktionsbedingungen entfernt.

Vorzugsweise werden freie, unmaskierte Aldehyde als Vorstufen verwendet, also Verbindungen der Formel (V), worin R⁵ = H ist. Die Synthese dieser Verbindungstypen kann in Analogie zu bekannten Verfahren vorgenommen werden.

Beispiele für die Herstellung dieser Monomere der Formel (V) sind Veresterung von vinylisch ungesättigten Säuren mit Hydroxyalkanalen, wie dieses in EP-A-003,516 beschrieben ist.

Dabei handelt es sich um Umsetzungen von Verbindungen der Formel (VIa) mit Verbindungen der Formel (VIb) 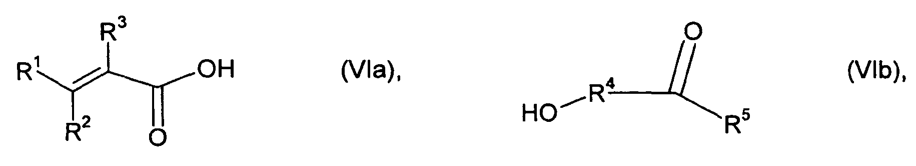 worin R¹, R², R³, R⁴ und R⁵ die weiter oben definierte Bedeutung besitzen.

Eine weitere Herstellung von Verbindungen der Formel (V) kann durch die Addition von Acrylsäure an Acrolein zu Formylethylacrylat erfolgen, wie dieses in JP-A 06072954, Beispiel 1A, beschrieben ist.

Eine weitere Herstellung von Verbindungen der Formel (V) kann durch Alkylierung von Salzen ungesättigter Carbonsäuren (VIIa) mit Halogenacetalen (VIIb) erfolgen, gefolgt von der Hydrolyse der erhaltenen Acetale zu den entsprechenden Aldehyden 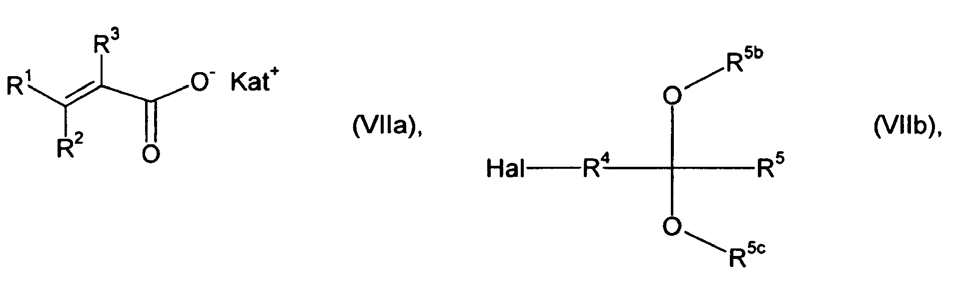 worin R¹, R², R³, R⁴, R⁵ und Kat⁺ die weitere oben definierte Bedeutung besitzen, Hal ein Halogenatom ist, vorzugweise Chlor oder Brom und R^{5b} und R^{5c} unabhängig voneinander Alkylreste bedeuten.

Ein Beispiel für eine derartige Reaktion ist die Alkylierung von Salzen der Methacrylsäure mit Halogenoacetalen, beispielsweise mit 2-Brom-1,1-diethoxyethan, zu 2,2-Diethoxyethylmethacrylat und nachfolgender Hydrolyse zu Formylethylmethacrylat, wie dieses von Zabranski et. al. in Makromol. Chem. 186, 215-222 (1985) beschrieben ist.

Eine weitere Herstellung von Verbindungen der Formel (V) kann durch die Umsetzung von Halogeniden ungesättigter Carbonsäuren (VIIa) mit Verbindungen der Formel (VIIIb) erfolgen, gefolgt von der Hydrolyse der erhaltenen Acetale zu den entsprechenden Aldehyden 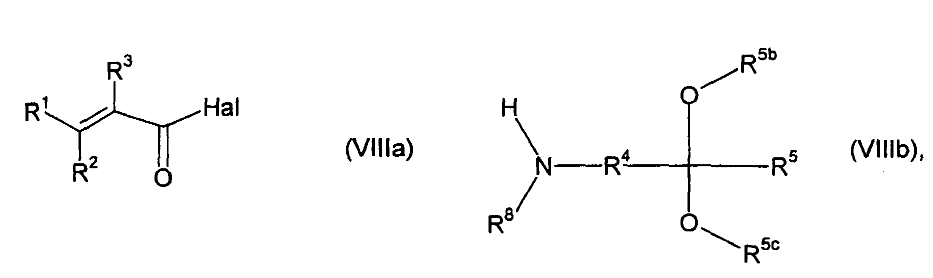 worin R¹, R², R³, R⁴, R⁵, R^{5b}, R^{5c}, R⁸ und Hal die weitere oben definierte Bedeutung besitzen.

Ein Beispiel für eine derartige Reaktion ist die Herstellung von N-(2,2-Dimethoxy-ethyl)-N-methylmethacrylamid aus N-(2,2-dimethoxyethyl)-N-methylamin und Methacryloylchlorid, wie dieses von Zabranski et. al. in Makromol. Chem. 186, 224 (1985) beschrieben ist.

Eine weitere Herstellung von Verbindungen der Formel (V) kann durch die Oxidation der Hydroxide der Formel (IX) mit Pyridiniumchromat erfolgen 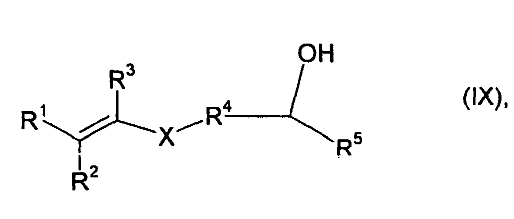 worin R¹, R², R³, R⁴, R⁵ und X die weitere oben definierte Bedeutung besitzen.

Ein Beispiel für eine derartige Reaktion ist die Oxidation von Hydroxyalkyl(meth)-acrylaten mit Pyridiniumdichromat, beispielsweise von Hydroxyethylacrylat zu Formylmethylacrylat, wie dieses von Luan et al. in Zhongguo Pige (China Leather) 32, 24-28 (2003) beschrieben ist.

Als besonders effektiv hat sich die TEMPO-katalysierte Oxidation von Hydroxyalkylacrylaten mit Hypochlorit zu Formylacrylaten (z.B. 3-Formylpropylacrylat aus Butandiolmonoacrylat, siehe Beispiel 2A) erwiesen. Diese relativ schonende Oxidation wurde von P. L. Anelli, F. Montanari und S. Quici in Organic Syntheses, Coll. Vol. 8, 367 (1993); Vol. 69, 212 (1990) beschrieben und führt in guten Ausbeuten zu den gewünschten Carbonylmonomeren.

Die Ausgangsprodukte zur Herstellung der Verbindungen der Formel (V) sind entweder bekannt und sind zum Teil kommerziell erhältlich oder können nach Standardverfahren der organischen Chemie hergestellt werden.

Aus den Vorstufen der Formel (V) können in bekannter Weise die erfindungsgemäßen Bisulfitaddukte (auch "Hydrogensulfitaddukte" genannt) hergestellt werden.

Grundsätzlich ist die Herstellung dieser Bisulfitaddukte an gesättigte und ungesättigte Aldehyde in der Literatur beschrieben. Es wird auf Houben-Weyl, Methoden der organischen Chemie, Band VII/I, Sauerstoffverbindungen II, Georg-Thieme- Verlag, Stuttgart 1954, S. 482-487, verwiesen.

Die Bisulfitaddukte können durch Umsetzung mit von schwefliger Säure abgeleiteten Salzen in einem leicht sauren Milieu gewonnen werden. Hierzu eignen sich Sulfite, Hydrogensulfite, Disulfite, oder Metabisulfite (Pyrosulfite) von Alkali- oder Erdalkalimetallen, oder auch von Ammonium, vorzugsweise von Natrium oder Kalium, oder man wählt die direkte Umsetzung mit schwefliger Säure oder durch eingeleitetes Schwefeldioxid. Es kann erforderlich sein, durch Auswahl geeigneter Puffersysteme einen für die Bildung der Bisulfitaddukte optimalen pH-Wert einzustellen.

Es ist bekannt, zur Vermeidung der konkurrierenden Addition an die C=C-Doppelbindung, bei höheren pH-Werten zu arbeiten. Im Rahmen dieser Untersuchung hat es sich als besonders zweckmäßig erwiesen, zur Vermeidung von Ausbeuteverlusten durch Bildung der Bisaddukte, die durch Addition an die C=C und C=O Gruppen entstehen können, die Salzverbindung unterstöchiometrisch zum eingesetzten Aldehyd einzusetzen und nicht umgesetzten Aldehyd zur Rezyklisierung zurückzugewinnen. Dadurch wird Hydrogensulfit ausschließlich an die reaktivere Carbonylgruppe addiert und die Reaktion in Richtung Zielverbindung dirigiert. Ferner hat es sich als zweckmäßig erwiesen, bei der Darstellung des Bisulfitaddukts weitgehend unter Sauerstoffausschluß zu arbeiten, um Sauerstoffinduzierte Redoxreaktionen, welche eine spontante Polymerisation auslösen können, zu unterdrücken. Die Vorgehensweise wird in den Ausführungsbeispielen näher erläutert.

Eine besonders bevorzugte Gruppe der vinylisch ungesättigten Bisulfitaddukte sind Formylalkylester und -amide von aliphatischen ethylenisch ungesättigten Mono-oder Dicarbonsäuren, insbesondere der Acrylsäure, der Methacrylsäure, der Fumarsäure, der Maleinsäure oder der Itaconsäure. Dabei handelt es sich um Verbindungen in Säure- oder Salzform, die ein Anion der Formel (X) und ein oder mehrere Kationen zur Herstellung der Elektroneutralität enthalten 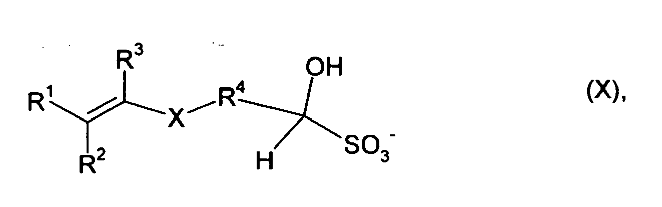 worin R¹, R² und R³ unabhängig voneinander Wasserstoff oder Methyl bedeuten, oder R¹ oder R² eine Gruppe der Formel -X-R ⁴-CH(OH)(SO3-) bedeuten, X -CO-O-oder -CO-NR⁸- ist, R⁸ Wasserstoff oder Methyl bedeutet, und R⁴ C₁-C₆-Alkylen, vorzugsweise Ethylen oder Propylen, ist.

Bei den Kationen zur Herstellung der Elektroneutralität handelt es sich vorzugsweise um ein- oder zweiwertige Kationen, vorzugsweise um ein Ammoniumkation, um ein Alkalimetallkation oder um ein Erdalkalimetalkation.

Besonders bevorzugt handelt es sich um Bisulfitaddukte von 2-Formylethylacrylat oder von 3-Formyl-propylacrylat. Dabei handelt es sich um gut isolierbare farblose und wasserlösliche Feststoffe, die in wäßriger Phase während der Polymerisation zudosiert werden können. Die Salze haben keinen definierten Schmelzpunkt, sondern zersetzen sich thermisch bei Temperaturen > 100 °C.

Die spektroskopischen Daten beider Verbindungen sind in den Beispielen aufgeführt und belegen die angenommene Konstitution.

Die Verbindungen der Formel (I) lassen sich als Vernetzermonomere bei der radikalischen Copolymerisation mit ethylenisch ungesättigten Comonomeren einsetzen.

Die Erfindung betrifft somit auch ein Copolymer abgeleitet von mindestens einem Monomer der Formel (I) und mindestens einem damit radikalisch copolymerisierbaren ethylenisch ungesättigten Comonomer.

Als Comonomere eignen sich im Prinzip sämtliche ethylenisch ungesättigten Monomeren, die sich mit den Monomeren der Formel (I) radikalisch polymerisieren lassen. Es können auch Gemische mehrerer Comonomerer eingesetzt werden.

Beispiele für ethylenisch ungesättigte Comonomere sind alpha-Olefine, aliphatische Kohlenwasserstoffe mit zwei oder mehreren konjugierten Doppelbindungen, Vinylester von gesättigten Carbonsäuren, Ester von ethylenisch ungesättigten Mono-oder Dicarbonsäure und/oder Alkenylaromaten.

Beispiele für alpha-Olefine sind einfach ethylenisch ungesättigte aliphatische Kohlenwasserstoffe mit zwei bis zehn Kohlenstoffatomen, besonders bevorzugt Ethylen und Propylen.

Beispiele für aliphatische Kohlenwasserstoffe mit zwei oder mehreren konjugierten Doppelbindungen sind Diene, insbesondere Buta-1,3-dien.

Beispiele für Vinylester von gesättigten Carbonsäuren sind Vinylester von aliphatischen gesättigten Carbonsäuren mit ein bis achtzehn Kohlenstoffatomen. Diese Comonomeren werden weiter unten ausführlicher beschrieben.

Beispiele für Ester von ethylenisch ungesättigten Mono- oder Dicarbonsäuren sind Alkylester von einfach ethylenisch ungesättigten C₃-C₈-Mono- oder Dicarbonsäuren, insbesondere von Acrylsäure, Methacrylsäure, Fumarsäure, Maleinsäure oder ltaconsäure. Diese Comonomeren werden weiter unten ausführlicher beschrieben.

Beispiele für Alkenylaromaten sind Vinylaromaten, wie Styrol oder alpha-Methylstyrol.

Die Herstellung der erfindungsgemäßen Copolymeren kann durch beliebige Arten der radikalischen Copolymerisation erfolgen.

Beispiele dafür sind die Polymerisation in Masse, die Polymerisation in Lösung, die Polymerisation in Suspension, in Dispersion, in Miniemulsion, in Mikroemulsion oder vorzugsweise die Polymerisation in Emulsion (,Emulsionspolymerisation"). Dem Fachmann sind diese Polymerisationstypen bekannt.

Dabei kann das erfindungsgemäße Vernetzermonomer als Bisulfitaddukt enthaltend ein Anion der Formel (I) bei der Copolymerisation eingesetzt werden. Dabei kann das fertige Bisulfitaddukt eingesetzt werden oder das Bisulfitaddukt wird in situ im Polymerisationsgemisch hergestellt. Alternativ kann aber auch ein Vorläufermonomer des Bisulfitadduktes bei der Copolymerisation eingesetzt werden, beispielsweise ein Aldehyd, und das entstandene Copolymere kann anschließend funktionalisiert werden, z.B. durch Zusatz von Bisulfit.

Das Vernetzermonomer der Formel (I) wird im allgemeinen nur in geringen Mengen eingesetzt, beispielsweise in Mengen bis zu 10 Gew. %, bezogen auf die Gesamtmenge an Monomer, vorzugsweise in Mengen von 0,01 bis 5 Gew. %.

Bei den erfindungsgemäßen Copolymerisaten handelt es sich bevorzugt um Copolymere enthaltend Struktureinheiten abgeleitet von Ethylen, Propylen, Styrol, Acrylat, Methacrylat, Vinylester gesättigter Carbonsäuren, Butadien oder von Gemischen von zwei oder mehreren dieser Monomeren sowie enthaltend bis zu 10 Gew. % an Struktureinheiten, bezogen auf Gesamtmonomer, die sich von Monomer der Formel (I) ableiten.

Besonders bevorzugte Copolymerisate liegen in Form von wässrigen Dispersionen vor und werden durch Emulsionspolymerisation hergestellt. Die Erfindung betrifft daher auch Zusammensetzungen in Form einer wässrigen Dispersion enthaltend ein Copolymerisat mit von Monomeren der Formel (I) abgeleiteten Struktureinheiten.

Die bevorzugten wässrigen Dispersionen sind im Wesentlichen auf Basis von ein oder mehreren ethylenisch ungesättigten Verbindungen aufgebaut, die sich von Vinylestern und/oder von Estem α, β-Ethylenisch ungesättigter C₃-C₈-Mono- oder Dicarbonsäuren und/oder von Alkenylaromaten ableiten sowie von den Monomeren der Formel (I).

Als Basis für die bevorzugten wässrigen Dispersionen kommen prinzipiell folgende Gruppen an Monomeren in Betracht:
Eine Gruppe bilden Vinylester von ein bis achtzehn Kohlenstoffatome aufweisenden Monocarbonsäuren, beispielsweise Vinylformiat, Vinylacetat, Vinylpropionat, Vinylisobutyrat, Vinylvalerat, Vinylpivalat, Vinyl-2-ethylhexanoat, Vinyldecanoat, Isopropenylacetat, Vinylester von gesättigten verzweigten Monocarbonsauren mit 5 bis 15 Kohlenstoffatomen im Säurerest, insbesondere Vinylester der Versatic™-Säuren, Vinylester von langerkettigen gesättigten oder ungesättigten Fettsäuren wie beispielsweise Vinyllaurat, Vinylstearat sowie Vinylester der Benzoesäure und substituierter Derivate der Benzoesäure wie Vinyl-p-tert.-butylbenzoat. Unter diesen ist jedoch Vinylacetat als Hauptmonomer besonders bevorzugt.

Eine weitere Gruppe von Monomeren, die neben den bevorzugten Vinylestern und/oder Estern der α, β-ethylenisch ungesättigten C₃-C₈-Mono- oder Dicarbonsäuren und/oder der Alkenylaromaten eingesetzt werden können, bilden aliphatische, monoolefinisch oder diolefinisch ungesättigte, gegebenenfalls halogen-substituierte Kohlenwasserstoffe, wie Ethen, Propen, 1-Buten, 2-Buten, Isobuten, konjugierte C₄-C₈-Diene, wie1,3-Butadien, Isopren, Chloropren, Vinylchlorid, Vinylidenchlorid, Vinylfluorid oder Vinylidenfluorid.

Eine weitere Gruppe von Monomeren für die bevorzugten wässrigen Dispersionen bilden Ester α, β-ethylenisch ungesättigter C₃-C₈-Mono- oder Dicarbonsäuren mit vorzugsweise C₁-C₁₈-Alkanolen und insbesondere C₁-C₈-Alkanolen oder C₅-C₈-Cycloalkanolen. Bei den Estern der Dicarbonsäuren kann es sich um Halbester oder vorzugsweise um Diester handeln. Geeignete C₁-C₈-Alkanole sind beispielsweise Methanol, Ethanol, n-Propanol, i-Propanol, 1-Butanol, 2-Butanol, Isobutanol, tert.-Butanol, n-Hexanol und 2-Ethylhexanol. Geeignete Cycloalkanole sind beispielsweise Cyclopentanol oder Cyclohexanol. Beispiele sind Ester der Acrylsäure, der Methacrylsäure, der Crotonsäure, der Maleinsäure, der Itaconsäure, Citraconsäure oder der Fumarsäure wie (Meth)acrylsäuremethylester, (Meth)acrylsäureethylester, (Meth)acryl-säure-isopropylester, (Meth)acrylsäure-n-butylester, (Meth)acrylsäureisobutyl-ester, (Meth)acrylsäure-1-hexylester, (Meth)acrylsäure-tert-butylester, (Meth)acrylsäure-2-ethylhexylester, Di-n-methylmaleinat oder -fumarat, Di-n-ethylmaleinat oder -fumarat, Di-n-propylmaleinat oder -fumarat, Di-n-butylmaleinat oder -fumarat, Diisobutyl-maleinat oder -fumarat, Di-n-pentylmaleinat oder -fumarat, Di-n-hexylmaleinat oder -fumarat, Dicyclohexyl-maleinat oder -fumarat, Di-n-heptylmaleinat oder -fumarat, Di-n-octylmaleinat oder -fumarat, Di-(2-ethylhexyl)maleinat oder -fumarat, Di-n-nonylmaleinat oder -fumarat, Di-n-decylmaleinat oder -fumarat, Di-n-undecylmaleinat oder -fumarat, Dilaurylmaleinat oder -fumarat , Dimyristiylmaleinat oder -fumarat, Dipalmitoylmaleinat oder -fumarat, Di-stearylmaleinat oder -fumarat und Diphenylmaleinat oder -fumarat.

Eine weitere Gruppe von bevorzugt eingesetzten Monomeren bilden die Alkenylaromaten. Dabei handelt es sich um Monoalkenylaromaten. Beispiele dafür sind Styrol, Vinyltoluol, Vinylxylol, α-Methylstyrol oder o-Chlorstyrol.

Die genannten Monomere bilden in der Regel die Hauptmonomeren, die in Bezug auf die Gesamtmenge der nach dem Verfahren der radikalischen wässrigen Polymerisation zu polymerisierenden Monomeren normalerweise einen Anteil von mehr als 50 Gew. %, bevorzugt mehr als 75 % auf sich vereinen.

Die Monomeren sind vorzugsweise so auszuwählen, dass ein Copolymerisat mit Klebstoff-, Bindemittel- oder Beschichtungseigenschaften entsteht. Dieses kann durch Einstellung der Glasübergangstemperatur der entstehenden Polymerisate in an sich bekannter Weise erfolgen.

Bevorzugte Hauptmonomere sind auf Basis folgender Polymerklassen aufgebaut:
Homo- oder Copolymere ein oder mehrerer Vinylester, insbesondere von Vinylacetat; Copolymere von Vinylestern mit Estern α, β-ethylenisch ungesättigter C₃-C₈-Mono- oder Dicarbonsäuren mit C₁-C₈-Alkanolen, insbesondere Ester der (Meth)acrylsäure und Malein-/oder Fumarsäure; Copolymere der Vinylester, insbesondere Vinylacetat, mit Ethen; Terpolymere aus Vinylestern, Ethen und Estern α, βethylenisch ungesättigten C₃-C₈-Mono- oder Dicarbonsäuren mit C₁-C₈-Alkanolen. insbesondere Estern der (Meth)acrylsäure und Malein-/oder Fumarsäure; Homo- oder Copolymere von Estern der (Meth)acrylsäure; Copolymere des Styrols mit Butadien und/oder Estern der α, β-ethylenisch ungesättigten C₃-C₈-Mono- oder Dicarbonsäuren mit C₁-C₈-Alkanolen, insbesondere Estern der (Meth)acrylsäure.

Selbstverständlich können weitere Comonomere, welche die Eigenschaften in gezielter Weise modifizieren, bei der Polymerisation mitverwendet werden. Solche Hilfsmonomere werden im Normalfall lediglich als modifizierende Monomere in Mengen, bezogen auf die Gesamtmenge der zu polymerisierenden Monomeren, von weniger als 50 Gew. %, in der Regel von weniger als 20, vorzugsweise zu weniger als 10 Gew. % einpolymerisiert.

Diese Monomeren dienen zur weiteren Stabilisierung der wässrigen Dispersionen, können durch Vernetzung während der Polymerisation oder während der Verfilmung die Filmkohäsion oder andere Eigenschaften verbessern und/oder durch eine geeignete Funktionalität mit Formulierungskomponenten unter Vernetzung reagieren.

Monomere, die zur weiteren Stabilisierung dienen können, sind in der Regel Monomere, die eine Säurefunktion aufweisen und/oder deren Salze. Zu dieser Gruppe zählen beispielsweise α,β-monoethylenisch ungesättigte Mono- und Dicarbonsäuren mit 3 bis 10 C-Atomen, ethylenisch ungesättigte Sulfonsäuren, ethylenisch ungesättigte Phosphonsäuren oder Dihydrogenphosphate und deren wasserlösliche Salze, z. B. deren Natriumsalze. Bevorzugte Monomere aus dieser Gruppe sind Vinylsulfonsäure und ihre Alkalisalze, Acrylamidopropansulfonsäure und ihre Alkalisalze, ethylenisch ungesättigte C₃-C₈-Carbonsäuren und C₄-C₈-Dicarbonsäuren, z. B. Itaconsäure, Crotonsäure, Vinylessigsäure, Acrylamidoglykolsäure und insbesondere Acrylsäure und Methacrylsäure.

Beispiele für weitere vernetzende Hilfsmonomere sind zwei oder mehr Vinylreste aufweisende Monomere, zwei oder mehr Vinylidenreste aufweisende Monomere sowie zwei oder mehr Alkenylreste aufweisende Monomere. Besonders vorteilhaft sind dabei die Di-Ester zweiwertiger Alkohole mit a,β-monoethylenisch ungesättigten Monocarbonsäuren, unter denen die Acryl- und Methacrylsäure bevorzugt sind, die Di-Ester zweiwertiger Carbonsäuren mit ethylenisch ungesättigten Alkoholen, sonstige Kohlenwasserstoffe mit zwei ethylenisch ungesättigten Gruppen oder die Di-Amide zweiwertiger Amine mit α,β-monoethylenisch ungesättigten Monocarbonsäuren.

Beispiele für derartige zwei nicht konjugierte ethylenisch ungesättigte Doppelbindungen aufweisende Monomere sind Alkylenglykoldiacrylate und -dimethacrylate, wie Ethylenglykoldiacrylat, 1,2-Propylenglykoldiacrylat, 1,3-Propylenglykoldiacrylat, 1,3-Butylenglykoldiacrylat, 1,4-Butylenglykoldiacrylate oder-methacrylate und Ethylenglykoldiacrylate oder-methacrylate, 1,2-Propylenglykoldimethacrylat, 1,3-Propylenglykol-dimethacrylat, 1,3-Butylenglykoldimethacrylat, 1,4-Butylenglykoldimethacrylate, Hexandioldiacrylat, Pentaerythroldiacrylat sowie Divinylbenzol, Vinylmethacrylat, Vinylacrylat, Vinylcrotonat, Allylmethacrylat, Allylacrylat, Diallylmaleat, Diallylfumarat, Diallylphthalat, Cyclopentadienylacrylat, Divinyladipat oder Methylenbisacrylamid.

Es können aber auch Monomere mit mehr als zwei Doppelbindungen eingesetzt werden, beispielsweise Tetraallyloxyethan, Trimethylolpropantriacrylat oder Triallylcyanurat.

Eine weitere Gruppe von Hilfsmonomeren ist geeignet, entweder durch Selbstvernetzung oder mit einem geeigneten monomeren Reaktionspartner und /oder mit den von Monomeren der Formel (V) abgeleiteten Struktureinheiten unter ausgewählten Bedingungen unter Vernetzung zu reagieren:
Zu dieser Gruppe gehören Monomere mit N-funktionellen Gruppen, darunter insbesondere (Meth)-acrylamid, Allylcarbamat, Acrylnitril, Methacrylnitril, Acrylamidoglykolsäure, Acrylamidomethoxyessigsäuremethylester, N-(2,2-Dimethoxy-1-hydroxyethyl)-acrylamid, N-Dimethylaminopropyl(meth)-acrylamid, N-Methyl-(meth)acrylamid, N-Butyl(meth)acrylamid, N-Cyclohexyl(meth)-acrylamid, N-Dodecyl(meth)acrylamid, N-Benzyl(meth)acrylamid, p-Hydroxyphenyl-(meth)-acrylamid, N-(3-Hydroxy-2,2-dimethylpropyl)methacrylamid, Ethylimid-azolidon-(meth)acrylat, N-(Meth)acryloyloxyethylimidazolidin-1-on, N-(2-Methacryl-amidoethyl)imidazolin-2-on, N-[[3-Allyloxy-2-hydroxypropyl]aminoethyl]imidazolin-2-on, N-Vinylformamid, N-Vinylpyrrolidon oder, N-Vinylethylenharnstoff.

Eine weitere Gruppe von Hilfsmonomeren bilden hyroxyfunktionelle Monomere, wie die Methacrylsäure- und Acrylsäure-C₁-C₉-Hydroxyalkylester, wie n-Hydroxyethyl-, n-Hydroxypropyl- oder n-Hydroxybutylacrylat und -methacrylat sowie deren Addukte mit Ethylenoxid oder Propylenoxid,

Eine weitere Gruppe von Hilfsmonomeren bilden solche, die über Carbonylgruppen vernetzbar oder selbstvernetzend sind. Beispiele sind Diacetonacrylamid, Allylacetoacetat, Vinylacetoacetat sowie Acetoacetoxyethylacrylat oder -methacrylat.

Eine weitere Gruppe von Hilfsmonomeren besteht aus Silangruppen enthaltenden Monomeren z. B. Vinyltrialkoxysilane, wie Vinyltrimethoxysilan, Vinyltriethoxysilan, Alkylvinyldialkoxysilane oder (Meth)acryloxyalkyltrialkoxysilane, z. B. (Meth)acryloxy-ethyltrimethoxysilan, oder(Meth)acryloxypropyltrimethoxysilan.

Eine weitere Gruppe von Hilfsmonomeren besteht aus epoxygruppenhaltigen Monomeren wie beispielsweise Allylglycidylether, Methacrylglycidylether, Butadienmonoepoxide, 1,2-Epoxy-5-hexen, 1,2-Epoxy-7-octen, 1,2-Epoxy-9-decen, 8-Hydroxy-6,7-epoxy-1-octen, 8-Acetoxy-6,7-epoxy-1-octen, N-(2,3-Epoxy)-propylacrylamid, N-(2,3-Epoxy)-propylmethacrylamid, 4-Acrylamidophenylglycidylether, 3-Acrylamidophenylglycidylether, 4-Methacrylamidophenylglycidylether, 3-Methacrylamidophenylglycidylether, N-Glycidoxymethylacrylamid, N-Glycidoxypropylmethacrylamid, N-Glycidoxyethylacrylamid, N-Glycidoxyethylmethacrylamid, N-Glycidoxy-propylacrylamid, N-Glycidoxypropylmethacrylamid, N-Glycidoxybutylacrylamid, N-Glycidoxybutylmeth-scrylamid, 4-Acrylamidomethyl-2,5-dimethylphenylglycidylether, 4-Methacrylamido-methyl-2,5-dimethyl-phenylglycidylether, Acrylamidopropyldimethyl-(2,3-epoxy)-propylammoniurnchlorid, Methacrylamidopropyldimethy-(2,3-epoxy)-propyl-ammoniumchlorid und Glycidylmethacrylat.

Neben dem Copolymeren enthalten die erfindungsgemäß bevorzugten wässrigen Dispersionen vorzugsweise Schutzkolloide. Dabei handelt es sich um polymere Verbindungen, welche während der Emulsionspolymerisation zugegen sind und die Dispersion stabilisieren.

Geeignete Schutzkolloide sind beispielsweise Polyvinylalkohole, Polyalkylenglykole, Alkalimetallsalze von Polyacrylsäuren und Polymethacrylsäuren, Cellulose-, Stärke- und Gelatinederivate oder von Acrylsäure, Methacrylsäure, Maleinsäure, Maleinsäureanhydrid, Methylvinylether, Styrol, 2-Acryl-amido-2-methylpropan-sulfonsäure und/oder 4-Styrolsulfonsäure abgeleitete Polymerisate und deren Alkalimetallsalze aber auch Polymere abgeleitet von N-Vinylpyrrolidon, N-Vinylcaprolactam, N-Vinylcarbazol, 1-Vinyl-imidazol, 2-Vinylimidazol, 2-Vinylpyridin, 4-Vinylpyridin, Acrylamid, Methacrylamid, amingruppentragenden Acrylaten, Methacrylaten, Acrylamiden und/oder Methacrylamiden. Eine ausführliche Beschreibung weiterer geeigneter Schutzkolloide findet sich in Houben-Weyl, Methoden der organischen Chemie, Band XIV/1, Makromolekulare Stoffe, Georg-Thieme-Verlag, Stuttgart, 1961, Seiten 411 bis 420.

Handelt es sich beim den Schutzkolloiden um Polyvinylalkohol, wird insbesondere Polyvinylalkohol vom Hydrolysegrad 60-100 Mol.-%, vorzugsweise 70 bis 98 Mol.-%, und Viskositäten der 4 Gew.-%igen wäßrigen Lösungen bei 20°C von 2 bis 70 mPa*s oder Gemische dieser Typen verwendet. Neben "homopolymerem" Polyvinylalkohol, d.h. nur aus Vinylalkohol- und restlichen Vinylacetatgruppen bestehendem Polyvinylalkohol, können copolymere bzw. funktionalisierte Polyvinylalkohole eingesetzt werden, beispielsweise Umsetzungsprodukte des Polyvinylalkohols mit Diketen oder mit Carboxylgruppen, Thiolgruppen, Formamidogruppen, Aminogruppen, Arylaminogruppen, Sulfatgruppen, Sulfonatgruppen, Phosphonatgruppen, quaternäre Ammoniumgruppen und andere funktionelle Gruppen tragende Typen von Polyvinylalkoholen.

Besonders bevorzugt werden in der wässrigen Dispersionen Schutzkolloide verwendet, die Komplexe oder koordinative Bindungen mit einer Verbindungsgruppe eingehen können, ausgewählt aus der Gruppe der sauren Metallsalze beziehungsweise Salze oder Säuren von Oxoanionen, insbesondere Aluminiumchlorid, Aluminiumnitrat, Titansulfat oder Zirkonoxychlorid, beziehungsweise Phosphorsäure oder Borsäure.

Die genannten polymeren Stabilisatoren können der wässrigen Dispersionen gegebenenfalls auch während oder nach der Polymerisation zugesetzt werden.

Bezogen auf den Feststoffanteil der wässrigen Dispersionen beträgt der Anteil der polymeren Schutzkolloide vorzugsweise 1 bis 35 Gew.-%, insbesondere 2 bis 20 Gew.-%.

Zusätzlich zu oder anstelle von den Schutzkolloiden kann die wässrige Dispersion auch mit Emulgatoren stabilisiert sein. Dabei kann es sich um ionische, vorzugsweise anionische, oder insbesondere um nichtionische Netzmittel handeln. Eine Auflistung geeigneter Emulgatoren finden sich in Houben-Weyl, Methoden der organischen Chemie, Band XIV/I, Makromolekulare Stoffe, Georg- Thieme-Verlag, Stuttgart, 1961, S. 192-208).

Der Anteil der Emulgatoren kann bis zu 10 Gew.-%, bezogen auf den Feststoffanteil der wässrigen Dispersion, betragen. Emulgatoren können bereits während der Polymerisation zugegen sein und/oder danach zugegeben werden.

Bevorzugt werden wässrige Dispersionen die Schutzkolloide enthalten und gegebenenfalls bis zu 2 Gew. %, bezogen auf den Feststoffanteil der Polymerdispersion an ionischen und/oder nichtionischen Emulgatoren.

Das Polymer und/oder das Schutzkolloid und/oder der Emulgator kann mit Aldehyden vernetzbare Gruppen aufweisen. Dabei handelt es sich beispielsweise um aktivierte Methylengruppen, Amino-, Amido-, Ureido und insbesondere um Hydroxylgruppen.

Besonders bevorzugt werden wässerige Dispersionen verwendet, die Schutzkolloide mit Hydroxylgruppen aufweisen, insbesondere Polyvinylalkohol und alkylierte Cellulosen.

Die erfindungsgemäßen wässrigen Dispersionen können weitere übliche Additive enthalten. Dazu zählen beispielsweise Filmbildehilfsmittel zur Erniedrigung der Mindestfilmbildetemperatur ("MFT-Erniedriger"), Weichmacher, Puffer, pH-Stellmittel, Dispergiermittel, Entschäumer, Füllstoffe, Farbstoffe, Pigmente, Silan-Kupplungsmittel, Verdickungsmittel, Viskositätsregler, Lösungsmittel und/oder Konservierungsmittel.

Eine Gruppe von Additiven stellen externe Vernetzungsmittel dar, welche in niedermolekularer Form bzw. als Vernetzerharze vorliegen können. Diese können den Effekt der Verbesserung der Wasserbeständigkeit in Kombination mit dem erfindungsgemäßen Vernetzer noch weiter verbessern und werden daher in den erfindungsgemäßen Zusammensetzungen besonders bevorzugt eingesetzt.

Als externe Vernetzungsmittel eignen sich beispielsweise Phenol-Formaldehyd-Harze, Resorcin-Formaldehyd-Harze, Melamin-Formaldehyd-Harze, Hydroxymethyl-substituierte Imidazolidinone oder Thioimidazolidinone, Hydroxymethyl-substituierte Pyrimidinone oder Hydroxymethyl-substituierte Triazinone oder Glycolurile oder deren Selbstkondensationsprodukte oder gemischte Kondensate aus zwei oder mehr der genannten Verbindungen, oder ein Gemisch aus zwei oder mehr der genannten Verbindungen. Beispielhaft genannt seien hierfür 1,3-bis(hydroxymethyl)-4-methoxy-4,5,5-trimethyl-2-Imidazolidinon, N,N'-Dimethylol-4-methoxy-5,5-dimethylpropyleneurea, N,N',N",N"'-Tetrakis(hydroxymethyl)glycoluril, 4,5-Dihydroxy-1,3-bis(methoxymethyl)-2-imidazolidinon,4,5-Dihydroxy-1,3-bis(hydroxymethyl)-imidazolidin-2-on, Tetrahydro-1,3-bis(hydroxymethyl)-4-methoxy-5,5-dimethyl-pyrimidin-2(1H)- on, 4,5-Dihydroxy-1,3-dimethylol-2-imidazolidinon, 4,5-Dihydroxy-1,3-dimethyl-2-imidazolidinon, Tetrahydro-1,3-bis(hydroxymethyl)-4-hydroxy-5,5-dimethyl-(1H)-pyrimidin-2-on, (=1,3-Dimethylol-4-methoxy-5,5-dimethylpropylenharnstoff), Tetrahydro-1,3-bis(hydroxymethyl)-4-alkoxy-5,5-dimethyl-(1H)-pyrimidin-2- on und N,N',N",N"'-Tetrakis(hydroxymethyl)glycoluril. Bevorzugt sind ebenfalls die in der EP-A 1 505 085 erwähnten partiell oder vollständig veretherten Harze auf Basis methylolierter Ethylenharnstoffe, Propylenharnstoffe, Glyoxaldiharnstoffe, Malondialdehyddiharnstoffe oder deren Kombinationen. Von diesen externen Vernetzungsmitteln werden diejenigen besonders bevorzugt verwendet, welche keine Hydroxymethylgruppen und somit keine Formaldehydquelle aufweisen.

Eine weiterhin hervorragend geeignete Gruppe von Additiven stellen andere Polyaldehyde wie Dialdehydstärken oder andere wasserlösliche Polyaldehyde, und ebenso die zumindest teilweise maskierten Polyaldehyde der EP-A-686,682 dar. Diese Verbindungen können in Kombination mit dem erfindungsgemäß modifizierten Copolymeren zu einer höheren Vernetzungsdichte beitragen.

Eine weiterhin hervorragend geeignete Gruppe von Additiven stellen Polyhydrazinderivate dar, insbesondere die in EP-A-3,516 genannten Verbindungen.

Besonders bevorzugt werden Zusammensetzungen enthaltend zusätzlich mindestens teilweise maskierte Polyaldehyde oder Polyhydrazinderivate.

Die erfindungsgemäße wässrige Dispersion besitzt vorzugsweise einen zur Abspaltung der Bisulfitgruppe geeigneten pH-Wert. Dabei kann es sich um sauer oder um basisch eingestellte Systeme handeln. In einer Ausführungsform weisen die erfindungsgemäßen Zusammensetzungen einen sauren pH-Wert auf. Dieser pH-Bereich liegt vorzugsweise zwischen 2 und 6, insbesondere zwischen 2,5 und 4,5. Ein geeigneter pH-Wert kann schon nach der Emulsionspolymerisation zur Herstellung der wässrigen Dispersion erreicht sein oder er kann durch Zugabe von sauren Verbindungen nachträglich eingestellt werden. Um den pH-Wert im angestrebten sauren Bereich einzustellen, sind organische oder anorganische Lewis- und Brφnsted-Säuren geeignet. Vorzugsweise geeignete Brφnsted-Säuren weisen einen pKₛ-Wert von <2,5 auf, beispielsweise Salzsäure, Schwefelsäure, Salpetersäure, Perchlorsäure, p-Toluolsulfonsäure, insbesondere Phosphorsäure. Als Lewis-Säure besonders geeignet sind die sauren Salze komplexierbarer Metallionen, insbesondere Aluminiumchlorid, Aluminiumnitrat, Zirkonoxychlorid und Titansulfat, insbesondere die sauren Salze mit mehrwertigen komplexierbaren Kationen, wie sie beispielsweise in DE-B 22 61 402, DE-C 26 20 738 und DE-A 39 42 628 aufgeführt sind.

Diese Produktgruppe der pH-Stellmittel ist vorzugsweise gleichzeitig zu Ausbildung von koordinativen Bindungen oder zur Komplexbildung mit den Schutzkolloiden der wässrigen Dispersionen befähigt. Hierzu eignen sich insbesondere die sauren Salze komplexierbarer Metallionen, insbesondere Aluminiumchlorid, Aluminiumnitrat, Zirkonoxychlorid und Titansulfat, insbesondere die sauren Salze mit mehrwertigen komplexierbaren Kationen, wie sie beispielsweise in DE-B 22 61 402, DE-C 26 20 738 und DE-A 39 42 628 aufgeführt sind.

Bevorzugt werden daher wässrige Dispersionen mit einem pH Wert von 2 und 6, insbesondere zwischen 2,5 und 4,5.

Der Feststoffgehalt der erfindungsgemäßen wässrigen Dispersionen beträgt vorzugsweise 20 bis 70 Gew.-%, insbesondere 30 bis 65 Gew.-%.

Die Herstellung der wässrigen Dispersionen kann nach den üblichen kontinuierlichen oder diskontinuierlichen Verfahrensweisen der radikalischen Emulsionspolymerisation erfolgen.

Die Durchführung einer radikalisch initiierten wäßrigen Emulsionspolymerisation von ethylenisch ungesättigten Monomeren ist vielfach vorbeschrieben und dem Fachmann daher hinreichend bekannt (vgl. z.B. Encyclopedia of Polymer Science and Engineering Vol. 8, Seiten 659 bis 677, John Wiley & Sons, Inc., 1987; D.C. Blackley, Emulsion Polymerisation, Seiten 155 bis 465, Applied Science Publishers, Ltd., Essex, 1975; D.C. Blackley, Polymer Latices, 2nd Edition, Vol. I, Seiten 33 bis 415, Chapman & Hall, 1997; H. Warson, The Applications of Synthetic Resin Emulsions, Seiten 49 bis 244, Ernest Bonn. Ltd.. London, 1972; D. Diederich, Chemie in unserer Zeit 1990, 24, Seiten 135 bis 142, Verlag Chemie, Weinheim; J. Piirma, Emulsion Polymerisation, Seiten 1 bis 287, Academic Press, 1982; F. Hölscher, Dispersionen synthetischer Hochpolymerer, Seiten 1 bis 160. Springer-Verlag, Berlin, 1969 und die Patentschrift DE-A 40 03 422). Sie erfolgt üblicherweise so, daß man die ethylenisch ungesättigten Monomeren, häufig unter Mitverwendung von Dispergierhilfsmitteln, in wäßrigem Medium dispers verteilt und mittels wenigstens eines radikalischen Polymerisationsinitiators polymerisiert.

Hierbei kommen wasserlösliche und/oder öllösliche Initiatorsysteme wie Peroxodisulfate, Azoverbindungen, Wasserstoffperoxid, organische Hydroperoxide oder Dibenzoylperoxid zum Einsatz. Diese können entweder für sich oder in Kombination mit reduzierenden Verbindungen wie Fe(II)-Salzen, Natriumpyrosulfit, Natriumhydrogensulfit, Natriumsulfit, Natriumdithionit, Natriumformaldehydsulfoxylat, Ascorbinsäure als Redoxkatalysatorsystem verwendet werden.

Die polymeren Schutzkolloide und/oder Emulgatoren können vor oder während der Polymerisation zugegeben werden. Eine zusätzliche Nachgabe von polymeren Stabilisatoren und/oder von Emulgatoren ist ebenfalls möglich. Zu dieser Dispersion werden dann gegebenenfalls noch für den gewünschten Anwendungszweck vorgesehene Additive zugegeben.

Die Formulierung der erfindungsgemäß bevorzugten wässrigen Dispersionen kann in den dafür dem Fachmann bekannten Vorrichtungen erfolgen, beispielsweise in Rührkesseln bzw. geeigneten Mixern. Ein Vermischen verschiedener Komponenten erst kurz vor der Applikation z. B. durch lnline-Sprühpistolen oder ähnliche Geräte ist ebenfalls möglich.

Die erfindungsgemäßen Copolymerzusammensetzungen lassen sich zu Produkten mit sehr hoher Wasserbeständigkeit verarbeiten ohne dass ein Risko der Formaldehydbildung in Kauf genommen werden muss.

Die erfindungsgemäßen Copolymerisate, insbesondere in Form von wässrigen Dispersionen, werden beispielsweise als Bindemittel in Zubereitungen eingesetzt, die zur Beschichtung von Substraten aller Art dienen. Hierunter fallen beispielsweise kunstharzgebundene Putze, Fliesenkleber, Dichtmassen und Versiegelungsmassen, vorzugsweise für poröse Bauteile, sowie Papierstreichmassen, Anstrichmittel, wie z.B. Dispersionsfarben, Dispersionslacke und Lasuren, sowie Bindemittel für

Nonwovens, Glasfasern und Fiberfill.

Besonders bevorzugt werden die erfindungsgemäßen Copolymerisate, insbesondere in Form von wässrigen Dispersionen, als Klebstoffe zum Verkleben beliebiger Substrate eingesetzt.

Diese Verwendungen sind ebenfalls Gegenstand der vorliegenden Erfindung

Besonders bevorzugt werden die erfindungsgemäßen Copolymersate, insbesondere in Form von wässrigen Dispersionen, zum Verkleben von porösen oder semiporösen Substraten oder als Bindemittel für poröse und semiporöse Substrat eingesetzt.

Eine spezielle Eignung der erfindungsgemäßen Copolymerisate, insbesondere in Form von wässrigen Dispersionen, liegt in der Anwendung als wasserresistenter Klebstoff insbesondere für cellulosische Substrate wie Holz, insbesondere Massivholz oder von Holz abgeleiteten Materialien und Werkstoffen, beispielsweise Furnieren, Sperrholz, Schichtholz, Lagenholz, Kunstharzpressholz, Verbundplatten oder Holzfaserwerkstoffen wie porösen, diffusionsoffenen, harten oder mitteldichten Holzfaserplatten (MDF) oder Kunststoff-beschichteten dekorativen Holzfaserplatten. Die Copolymerisate eignen sich für die manuelle oder maschinelle Applikation sowie insbesondere auch für Anwendungen, in denen die Klebfugen durch hochfrequente Wechselströme gehärtet werden.

Weitere generelle Anwendungsbeispiele sind wasserfeste Verklebungen von Papier, Pappe, Wellpappe, Schaumstoff, Zement, Leder, Textil oder Preßschichtstoffen.

Andere Anwendungen liegen in Klebstoffen für den Baubereich als Fußboden-, Wand- oder Deckenklebstoff oder als Möbelfolien- oder Teppichrückenkleber.

Weitere Eignungsgebiete liegen in wasserfesten Bindemitteln für Holzfaserplatten oder Faserleder sowie Bindemittel für Dämm-Materialien aus Papier- oder Kunststoff-Fasern, ferner in wasserresistenten Baustoffdispersionen als Bindemittel für Putz oder Zement.

Ein weiteres Einsatzgebiet für die erfindungsgemäßen Copolymerisate, insbesondere in Form von wässrigen Dispersionen, sind Bindemittel für Textil und Non-Woven (sog. Engineered Fabrics) sowie im Textildruck und als Textilappretur.

Ein bevorzugtes Einsatzgebiet besteht in der Verwendung als Bindemittel für Glasfasern, welche beispielsweise zur Verfestigung von Kunststoff-Fliesen, Formkörpern und als Dämmmaterial verwendet werden oder als Bindemittel für Keramik.

Ein weiteres bevorzugtes Einsatzgebiet besteht in der Verwendung als Bindemittel für Anstrichmittel, insbesondere für Dispersionsfarben, Dispersionslacke und Lasuren.

Die erfindungsgemäßen Zusammensetzungen können auch zur Herstellung von redispergierbaren Dispersionspulvern eingesetzt werden. Diese können in an sich bekannter Weise durch Versprühen der wässrigen Dispersionen erzeugt werden.

Die nachfolgenden Beispiele dienen der Erläuterung der Erfindung. Die in den Beispielen angegebenen Teile und Prozente beziehen sich auf das Gewicht, soweit nicht anders vermerkt.

### Beispiel 1A: Darstellung von 2-Formylethylacrylat in Anlehnung an JP-A-60/72,954

In einem zylindrischen Glasreaktor von 3000 ml Inhalt, versehen mit Rührer und Dosiervorrichtungen, wurden 159,6 g (2,85 mol) Acrolein (Qualität > 95 %, Fluka) zusammen mit 100 mg Hydrochinon vorgelegt. Unter Rühren wurden dann 1024 g (14,2 mol) Acrylsäure (BASF AG) und anschließend 66,6 g Amberlyst A 21 (basischer Ionenaustauscher der Firma Rohm & Haas) zugegeben. Das Gemisch wurde im Wasserbad auf 50 °C Innentemperatur erwärmt, bei dieser Temperatur für 10 Stunden gerührt und anschließend auf Raumtemperatur abgekühlt. Zur Isolierung des Rohprodukts wurde die abgekühlte Reaktionsmischung über einen Faltenfilter filtriert und das Filtrat im Rotationsverdampfer unter Wasserstrahlvakuum bei 72°C Badtemperatur so lange eingeengt bis kein Destillat mehr überging. Der Eindampfrückstand wurde in 400 ml Dichlormethan aufgenommen und 8 mal mit je 400 ml 5 %iger Natriumbicarbonatlösung im Becherglas schnell gerührt, sowie anschließend mit 2 mal 400 ml E-Wasser ausgeschüttelt. Die organische Phase wurde über 20 g Magnesiumsulfat getrocknet und das Methylenchlorid anschließend im Rotationsverdampfer abdestilliert. Der Rückstand (35,8 g) wurde im Vakuum destilliert. Die Hauptfraktion wurde im Siedebereich 79-81 °C (P < 2 mm Hg) erhalten.
Ausbeute: 12 g (3,3 % bezogen auf eingesetztes Acrolein).

Das IR-Spektrum der Verbindung war identisch mit dem in JP-A-60/72,954 auf Seite 4 publiziertem Spektrum.

### Beispiel 1B: Darstellung des Bisulfitaddukts von 2-Formylethylacrylat (FEA-BSA):

12 g (0,094 mol) 2-Formylethylacrylat wurden in einem mit Stickstoff überspülten 100 ml Erlenmeyerkolben vorgelegt und mit 0,076 g (0,5 mmol) 1-Phenyl-3-pyrazolidinon (als Stabilisator) versetzt. Anschließend wurde eine Lösung von 5,34 g (0,028 mol) Natriummetabisulfit in 117 ml entionisiertem Wasser unter guter Durchmischung und Wasserkühlung (ca. 20 °C) über 15 Minuten zur Reaktionsmischung getropft. Nach beendeter Zugabe wurde für weitere 10 Minuten gerührt. Anschließend wurde die Reaktionsmischung mit 2 x ca. 30 ml Ethylacetat zur Rückgewinnung von nicht umgesetztem 2-Formylethylacrylat extrahiert (siehe unten). Zur Fällung des Addukts wurde die wäßrige Phase zunächst im Eisbad mit 12 ml Ethanol versetzt und gerührt bis das Produkt ausfiel. Anschließend wurden ca. 30 ml im Eisbad vorgekühltes Ethylacetat zur Reaktionsmischung gegeben und der feinkristalline Niederschlag aufgeschlämmt. Das Produkt wurde über eine Nutsche abfiltriert, mit dem restlichen Ethylacetat gewaschen und im Vakuumtrockenschrank getrocknet. Es wurden 5,2 g (22,5 %), bezogen auf eingesetzten Aldehyd, erhalten.
¹H-NMR (D₂O; RT; ppm): 6.45 *H*_{cis}HₜᵣₐₙₛC=CH- (dd, *J* = 17.4, 1.1 Hz, 1H), 6.22 H_{cis}HₜᵣₐₙₛC=C*H*- (dd, *J* = 17.4, 10.6 Hz, 1 H), 6.00 H_{cis}*H*ₜᵣₐₙₛC=CH- (dd, *J* = 10.6, 1.1 Hz, 1H), 4.56 HO-C(*H*)SO₃Na- (m, 1H), 4.38 -O-C*H*₂- (m, 2H), 2.38, 2.05 -C*H*_{*A*/*B*}-C(H)(OH)SO₃⁻ (m, 1 H; m 1 H).
¹³_{H}-NMR (D₂O; RT; ppm): 171.1 -C(=O)-O-, 135.3 H₂*C*=, 130.2 =CH-, 83.5 - *C*(H)(OH)-SO₃⁻, 64.2 -O-*C*H₂-, 32.9 -*C*H₂-C(H)(OH)SO₃⁻
IR (KBr, cm⁻¹): 3416 br. s, 2964 w, 1726 s, 1636 m, 1619 w, 1412 m, 1299 m, 1199 s, 1123 m, 1044 s, 987 m, 812 m, 635 m, 585 m, 536 m, 436 w.

### Beispiel 2A: Darstellung von 3-Formylpropylacrylat durch Oxidation von Butandiolmonoacrylat

Die nachfolgend beschriebene Reaktion wurde in Anlehnung an P. L. Anelli, F. Montanari, S. Quici; Organic Syntheses, Coll. Vol. 8, .367 (1993); Vol. 69, 212 (1990) ausgeführt.

In einem 2 I Dreihalsrundkolben mit Magnetrührer und Innenthermometer wurden 96,1 g (0,667 mol) Butandiolmonoacrylat (Handelsware der BASF AG) und 1,17 g (7,51 mmol) TEMPO (2,2,6,6-Tetramethylpiperidin-N-oxyl) eingewogen und in 340 g Methylenchlorid gelöst. Hierzu wurde eine Lösung aus 8,94 g (75,1 mmol) KBr in 37,5 ml entionisiertem Wasser gegeben und unter intensiver Durchmischung mittels einer geeigneten, wirksamen Kühlvorrichtung auf 0 - 10°C abgekühlt. In einem separaten Gefäß wurden 614 g (ca. 0,825 mol) einer wässrigen ca. 10 %igen Natriumhypochloritlösung mit 200 ml entionisiertem Wasser verdünnt und mit 25,5 g (0,304 mol) Natriumbicarbonat auf pH 9 eingestellt (der pH ist mit einer geeigneten Messvorrichtung zu überprüfen; benötigte Menge Natriumbicarbonat kann je nach verwendeter Natriumhypochloritlösung abweichen) und anschließend über einen Tropftrichter innerhalb von 15-20 Minuten zur Reaktionsmischung getropft. Die Temperatur wurde dabei nach Möglichkeit (Kühlung) knapp über 0°C gehalten und sollte 15°C nicht übersteigen. Nach erfolgter Zugabe wurde für weitere 3 Minuten gerührt. Nach beendeter Reaktion wurden die organische und die wässrige Phase möglichst schnell voneinander getrennt und die wässrige Phase wurde mit 260 g Methylenchlorid extrahiert. Die vereinigten organischen Phasen wurden anschließend nacheinander mit einer Lösung von 2,40 g (14,5 mmol) Kaliumiodid in 150 ml 10 %iger wässriger Salzsäure, einer Lösung aus 9,9 g (62,6 mmol) Natriumthiosulfat in 90 ml Wasser, 2 x 150 ml einer 10%igen Natriumbicarbonatlösung in entionisiertem Wasser und zuletzt mit 150 ml entionisiertem Wasser gewaschen. Die organische Phase wurde über wasserfreiem Magnesiumsulfat getrocknet und anschließend das Lösungsmittel am Rotationsverdampfer entfernt. Es wurden 89 g Rohprodukt erhalten.
Als Hauptverunreinigungen waren im Rohprodukt noch ca. 6 mol % Butandiolmonoacrylat sowie ca. 1 mol % eines Halbacetals aus 3-Formylpropylacrylat und Butandiolmonoacrylat enthalten. Das Rohprodukt kann jedoch ohne weitergehende Aufreinigung zur Darstellung des Bisulfitaddukts verwendet werden.
¹H-NMR (CDCl₃; RT; ppm): 9.80 -C*H*=O (t, *J* = 1.2 Hz, 1H), 6.39 *H*_{cis}HₜᵣₐₙₛC=CH-(dd, *J* = 17.5, 1.5 Hz, 1H), 6.11 H_{cis}HtᵣₐₙₛC=C*H*- (dd, *J* = 17.5, 10.5 Hz, 1H), 5.84 H_{cis}*H*ₜᵣₐₙₛC=CH- (dd, *J* = 10.5, 1.5 Hz, 1 H), 4.20 -O-C*H*₂- (t, *J* = 6.5 Hz, 2H), 2.57 - C*H*₂-CH=O (td, *J* = 7.0, 1.2 Hz, 2H), 2.03 -CH₂-C*H*₂-CH₂- (tt, *J* = 7.0, 6.5 Hz, 2H).
¹³H-NMR (CDCl₃; RT; ppm): 201.1 -*C*H=O, 166.0 -*C*(=O)-O-, 130.9 H₂*C*=, 128.3 =*C*H-, 63.5 -O-*C*H₂-, 40.5 -*C*H2-CH=O. 21.4 -CH₂-*C*H₂-CH₂-.
IR (Film auf KBr, cm⁻¹): 3107 w, 3039 w, 2962 m, 2900 m, 2832 m, 2728 m, 1724 s, 1636 m, 1620 m, 1457 m, 1442 m, 1410 s, 1297 s, 1273 s, 1192 s, 1063 s, 986 s, 811 s, 667 w.

### Beispiel 2B: Bisulfitaddukt von 3-Formylpropylacrylat (FPA-BSA):

133 g (0,93 mol) 3-Formylpropylacrylat wurden in einem mit Stickstoff überspülten 250 ml 2-Halskolben vorgelegt und mit 0,76 g (4,7 mmol) 1-Phenyl-3-pyrazolidinon (als Stabilisator) versetzt. Anschließend wurde eine Lösung von 53,2 g (0,28 mol) Natriummetabisulfit in 117 ml entionisiertem Wasser unter guter Durchmischung und Wasserkühlung (ca. 20 °C) über 15 Minuten zur Reaktionsmischung getropft. Nach beendeter Zugabe wurde für weitere 10 Minuten gerührt. Anschließend wurde die Reaktionsmischung mit 2 x ca. 100 ml Ethylacetat zur Rückgewinnung von nicht umgesetztem 3-Formylpropylacrylat extrahiert (siehe unten). Anschließend wurde die wässrige Phase in einem Eisbad mit 100 Ethanol versetzt bis zur Kristallisation des Produkts gerührt. Anschließend wurde der Kristallbrei ebenfalls im Eisbad mit ca. 500 ml vorgekühltem Ethylacetat versetzt, aufgeschlämmt und anschließend filtriert. Der Filterkuchen aus 3-Formylpropylacrylat wurde mit 2 x 300 ml Ethylacetat gewaschen und getrocknet. Es wurden 82 g (36 % bezogen auf Aldehyd; 60 % bezogen auf Natriummetabisulfit) erhalten.

Zur Rückgewinnung von nicht umgesetztem 3-Formylpropylacrylat wurde die Ethylacetatphase über wasserfreiem Magnesiumsulfat getrocknet und das Methylenchlorid im Vakuum entfernt. Das Rezyklat enthielt noch unbekannte Verunreinigungen, konnte aber problemlos als 50/50 Mischung mit frischem Aldehyd zur Darstellung des Bisulfitaddukts von 3-Formylpropylacrylat eingesetzt werden.
¹H-NMR (d6-DMSO; RT; ppm): 6.32 *H*_{cis}HₜᵣₐₙₛC=CH- (dd, *J* = 17.3, 1.6 Hz, 1H), 6.17 H_{cis}HₜᵣₐₙₛC=C*H*- (dd, *J* = 17.3, 10.3 Hz, 1H), 5.93 H_{cis}*H*ₜᵣₐₙₛC=CH- (dd, *J* = 10.3, 1.6 Hz, 1H), 5.23 *H*O-C(H)SO₃⁻ (d, *J* = 5.7 Hz, 1H), 4.10 -O-C*H*₂- (m, 2H), 3.82 HO-C(*H*)SO₃Na- (m, 1 H), 1.80 -CH₂-C*H*₂-CH₂- (m, 2H), 1.65, 1.52 -C*H*_{*A*/*B*}-C(H)(OH)SO3⁻ (m, 1H; m 1 H).
¹³H-NMR (d6-DMSO; RT; ppm): 169.3 -*C*(=O)-O-, 131.3 H₂*C*=, 128.3 =*C*H-, 82.3 -*C*(H)(OH)-SO3-, 64.1 -O-*C*H₂-, 28.0 / 24.8 *-*CH₂-CH₂-C(H)(OH)SO₃⁻

IR (KBr, cm⁻¹): 3352 br. s, 3040 w, 2960 m, 2902 w, 1729 s, 1634 m, 1620 w, 1467 m, 1409 s 1390 m, 1362 m, 1296 s, 1272 s, 1246 s, 1230 s, 1212 s, 1189 s, 1168 s, 1151 s, 1122 m, 1098 m, 1046 s, 986 s, 885 w, 809 m, 765 w, 675 m, 632 s.

### Polymerisationen mit den Bisulfit-Addukten FEA-BSA und FPA-BSA

Alle Mengenangaben in "Teilen" verstehen sich als Masse der jeweiligen Substanz bezogen auf die Masse des eingesetzten Vinylacetats.

**Allgemeine Vorschrift**: In einem mit Ankerrührer, Zulaufmöglichkeiten, Rückflusskühler, Mantelheizung und -kühlung versehenen Glasrührkesselreaktor wurde eine Lösung von 10 Teilen teilverseifter Polyvinylalkohol mit einem Hydrolysegrad von 88 Mol % und einer Viskosität der 4 Gew.-%igen Lösung bei 20 °C von 18 mPas in 88 Gewichtsteilen entionisiertem Wasser hergestellt. Nach Zugabe von 0,09 Teilen Entschäumungsmittel ®Agitan 280 (Münzing-Chemie) und 0,1 Teilen Natriumacetat wurden 8,8 von insgesamt 100 Teilen Vinylacetat einemulgiert. Die Innentemperatur wurde auf 60°C angehoben und die Polymerisation durch die Zugabe einer Lösung aus 0,02 Teilen Ammoniumpersulftat in 0,66 Teilen entionisiertem Wasser gestartet.

Nach dem Anspringen der Reaktion wurden innerhalb von 3 Stunden 91,2 Teile Vinylacetat, eine Lösung aus 0,03 Teilen Ammoniumpersulfat in 2,5 Teilen entionisiertem Wasser und eine Lösung der in der Tabelle 1 aufgeführten funktionellen Comonomere in 2,5 Teilen entionisiertem Wasser in drei getrennten Zuläufen gleichmäßig in den Polymerisationsansatz eindosiert. Die Manteltemperatur wurde so gesteuert, dass die Polymerisation bei einer langsam steigenden Innentemperatur von 68°C am Anfang bis ca. 80°C am Ende der Dosierung verlief.

Nach beendeter Dosierung wurde eine Lösung aus 0,01 Teilen Ammoniumpersulfat in 0,5 Teilen entionisiertem Wasser zugegeben und zunächst eine Stunde bei 80°C nachpolymerisiert.

Im Falle der Beispiele 3 und V1 wurde zur Entmonomerisierung mit Lösungen von 0,04 Teilen Trigonox AW 70 (Fa. Akzo, tert.Butylhydroperoxid) in 0,15 Teilen Wasser und 0,07 Teilen Ascorbinsäure in 0,5 Teilen Wasser bei 80 °C bzw. 75 °C nachpolymerisiert. Bei den Beispielen 4-7 erfolgte die Entmonomerisierung mit Lösungen von 0,08 Teilen Natriummetabisulfit in 0,5 Teilen entionisiertem Wasser und 0,05 Teilen Ammoniumpersulfat in 0,5 Teilen entionisiertem Wasser bei 80 °C / 75 °C. Zusätzlich wurden bei diesen Beispielen noch jeweils Lösungen von 0,2 Teilen Natriummetabisulfit in 2 Teilen entionisiertem Wasser zur Stabilisierung bei Raumtemperatur eingerührt. Man erhielt so Dispersionen mit Feststoffgehalten zwischen 51 und 53 %. Die Viskositäten der erhaltenen Produkte sind in Tabelle 2 aufgeführt.

Für die Ausprüfung als Holzklebstoff wurden die Produkte noch mit jeweils 2 Teilen Butyldiglycolacetat, 0,025 Teilen Entschäumer ®Agitan 305 (Münzing-Chemie) sowie 5 Gewichtsteilen einer 28 %igen wässrigen Aluminiumchloridlösung modifiziert.

Die Ausprüfung der formulierten Dispersionen erfolgte an Buchenholzprüflingen gemäß der Prüfnorm DIN EN 204/D3. In diesem Test wird die Beständigkeit des Klebstoff-Films gegenüber viertägiger Kalt-Wasserbelastung getestet. Ohne Einsatz von vernetzenden Monomeren würde der Klebstoff-Film keinerlei Wasserbeständigkeit, besitzen, d.h. die Prüfkörper würden während der Lagerung auseinanderfallen. Neben der prinzipiellen Aussage über die anwendungstechnische Eignung der Produkte in der gewählten Applikation gibt der Test auch gleichzeitig einen Hinweis über die Vernetzungsdichte im Film, da die Werte der Naßklebefestigkeit mit zunehmender Film-Quellung und Reemulgierbarkeit abnehmen, welche durch Vernetzung effektiv reduziert werden. Dies geht auch aus den Wasseraufnahmen der reinen Filme hervor, die durch eine gängige Methode (siehe EP-B 1 458 774 S. 8) bestimmt wurden. Die Verleimung und Prüfung wurde unter Berücksichtigung folgender Kenndaten durchgeführt:

**Tabelle 1 - Parameter der Normverklebung**

| | |
|---|---|
| Leimauftrag | 150±20 g/m² (beidseitiger Auftrag) |
| Preßzeit | 2 Stunden |
| Preßdruck | 0,7 N/mm² |
| Anzahl der Prüfkörper pro Prüffolge | 20 |
| Prüfung nach Lagerungsfolge | 7 Tage bei Normalklima |
| gemäß DIN EN 204 D3/3; sofortige | 4 Tage in kaltem Wasser |
| Prüfung | |
| Prüftemperatur | 23±2°C |
| Vorschubgeschwindigkeit | 50 mm/Min. |
| Geforderte Reißfestigkeit | ≽ 2 N/mm² |

In Tabelle 2 sind die Ergebnisse der Polymerisationen und die anwendungstechnischen Testergebnisse aufgeführt. Bei Vergleichsbeispiel V1 wurde zum Vergleich eine Polymerisation mit dem üblichen Vernetzermonomer N-Methylolacrylamid durchgeführt. Hierzu wurde eine handelsübliche 48 %ige Ware der Firma S.N.F. Floerger verwendet. Die in der Tabelle angegebene Menge bezieht sich auf die Aktivsubstanz. In den Beispielen 3 und 4 wurden jeweils zu dieser Menge molar äquivalente Mengen der Bisulfitadduktmonomeren verwendet, um einen direkten Vergleich der Vernetzungseffizienz ziehen zu können. Alle Formulierungen der erfindungsgemäßen Beispieldispersionen ergaben einen nicht reemulgierenden Film. Der beobachtete Vernetzungseffekt reicht zum sicheren Bestehen der gewählten Prüfnorm aus. Man sieht, daß bei äquimolarem Ersatz von N-Methylolacrylamid durch die neuen Monomere die Effizienz vergleichbar, oder wie im Falle von FPA-BSA, sogar besser ist. Formaldehyd wird weder durch die Produkte eingetragen, noch entsteht er während der Vernetzung.

**Tabelle 2 - Daten der Polymerisations-Versuche und Tests**

| Beispiel | Vernetzer-monomer | Verwendete Menge (auf 100 Teile Vinylacetat) | Viskosität Brookfield RVT, 23 °C [mPa*s] | Wasseraufnahme des Films [%] nach Formulierung | Nass-Klebfestigkeit auf Buchenholz gemäß EN 204 D3/3 [N/mm²] nach Formulierung |
|---|---|---|---|---|---|
| 3 | FEA-BSA | 0,88 | 11.600 | 27 | 2,6 |
| 4 | FPA-BSA | 0,93 | 120.000 | 14 | 7,0 |
| 5 | FPA-BSA | 0,7 | 41.000 | 22 | 3,9 |
| 6 | FPA-BSA | 0,47 | 26.400 | 32 | 2,6 |
| 7 | FPA-BSA | 0,23 | 19.950 | 35 | 1,5 |
| V1 | NMA | 0,38 | 28.750 | - | 2,3 |

## Patentansprüche

1. Copolymerisat abgeleitet von mindestens einem Monomer in Säure- oder Salzform, das ein Anion der Formel (I) und ein oder mehrere Kationen zur Herstellung der Elektroneutralität enthält, und mindestens einem weiteren damit radikalisch copolymerisierbaren Monomer 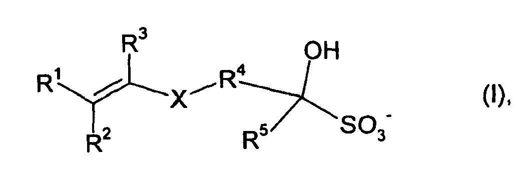 worin
R¹ und R² unabhängig voneinander Wasserstoff, Alkyl, Cycloalkyl, Aryl, Aralkyl, -COOR⁵, -COO⁻ Kat⁺ oder -CON(R⁶R⁷) bedeuten,
R⁶ und R⁷ unabhängig voneinander Wasserstoff, Alkyl oder Aryl sind,
Kat⁺ ein einwertiges Kation bedeutet, und
einer der Reste R¹ oder R² auch eine Gruppe -X-R⁴-CR⁵(OH)(SO₃) bedeuten kann,
wobei Kat⁺ ein einwertiges Kation ist und X, R⁴ und R⁵ eine der unten genannten Bedeutungen annehmen,
R³ Wasserstoff, Alkyl oder Aryl bedeutet,
X ausgewählt wird aus der Gruppe direkte C-C-Bindung, -O-, -CH₂-O-, -CH₂-NR⁸-, -COO- oder -CONR⁸-,
R⁸ = Wasserstoff, Alkyl oder Aryl bedeutet,
R⁴ Alkylen, Polyoxyalkylen, Cycloalkylen oder Arylen bedeutet, und
R⁵ Wasserstoff, Alkyl, Cycloalkyl oder Aryl bedeutet.

2. Copolymerisat nach Anspruch 1, **dadurch gekennzeichnet, dass** R¹, R² und R³ unabhängig voneinander Wasserstoff oder Methyl und einer der Reste R¹ oder R² -COOR^{5a} oder -COO⁻ Kat⁺ bedeuten kann, wobei R^{5a} Wasserstoff oder C₁-C₆-Alkyl ist.

3. Copolymerisat nach Anspruch 1, **dadurch gekennzeichnet, dass** R⁴ Alkylen oder Polyoxyalkylen bedeutet, bevorzugt unsubstituiertes C₁-C₆-Alkylen, und ganz besonders bevorzugt unsubstituiertes C₁-C₄-Alkylen.

4. Copolymerisat nach einem der Ansprüche 1 bis 3, **dadurch gekennzeichnet, dass** die Kationen zur Herstellung der Elektroneutralität ausgewählt werden aus der Gruppe der ein- bis vierwertige Kationen, vorzugsweise des Wasserstoffs, des Amoniums oder der ein- bis vierwertigen Metallionen.

5. Copolymerisat nach Anspruch 1, **dadurch gekennzeichnet, dass** das Anion der Formel (I) ein Anion der Formel (IVa) oder (IVb) ist 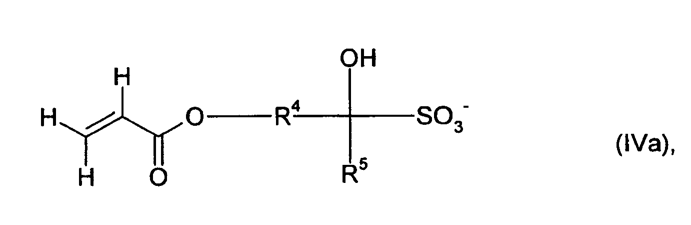 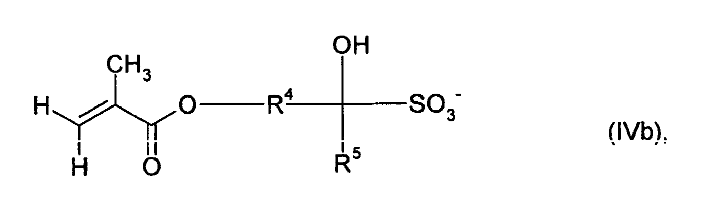 worin R⁴ und R⁵ die in Anspruch 1 definierte Bedeutung besitzen.

6. Copolymerisat nach Anspruch 1, **dadurch gekennzeichnet, dass** das weitere radikalisch copolymerisierbare Monomer ausgewählt wird aus der Gruppe der alpha-Olefine, der aliphatischen Kohlenwasserstoffe mit zwei oder mehreren konjugierten Doppelbindungen, der Vinylester von gesättigten Carbonsäuren, der Ester von ethylenisch ungesättigten Mono- oder Dicarbonsäure und/oder der Alkenylaromaten.

7. Copolymerisat nach Anspruch 6, **dadurch gekennzeichnet, dass** das Copolymere Struktureinheiten enthält, die sich ableiten von Ethylen, Propylen, Styrol, Acrylat, Methacrylat, Vinylester gesättigter Carbonsäuren, Butadien oder von Gemischen von zwei oder mehreren dieser Monomeren sowie von bis zu 10 Gew. % an Struktureinheiten, bezogen auf Gesamtmonomer, die sich vom Monomer der Formel (I) ableiten.

8. Zusammensetzung enthaltend ein Copolymerisat nach Anspruch 1 in Form einer wässrigen Dispersion.

9. Zusammensetzung nach Anspruch 8, **dadurch gekennzeichnet, dass** diese mindestens einen Polyaldehyd oder mindestens ein Polyhydrazin enthält.

10. Zusammensetzung nach einem der Ansprüche 8 und 9, **dadurch gekennzeichnet, dass** diese mindestens einen zumindest teilweise maskierten Polyaldehyd enthält.

11. Zusammensetzung nach einem der Ansprüche 8 bis 10, **dadurch gekennzeichnet, dass** diese Lewis-Säuren in Form von sauren Salzen komplexierbarer Metallionen enthält, insbesondere Aluminiumchlorid, Aluminiumnitrat, Zirkonoxychlorid und Titansulfat, und ganz besonders die sauren Salze mit mehrwertigen komplexierbaren Kationen.

12. Zusammensetzung nach einem der Ansprüche 8 bis 11, **dadurch gekennzeichnet, dass** diese einen pH Wert von 2,5 bis 4,5 aufweist.

13. Gehärtete Zusammensetzung erhältlich durch Auftragen und Trocknen einer Zusammensetzung nach einem der Ansprüche 8 bis 12.

14. Verbindung in Säure- oder Salzform, die ein Anion der Formel (I) und ein oder mehrere Kationen zur Herstellung der Elektroneutralität enthält 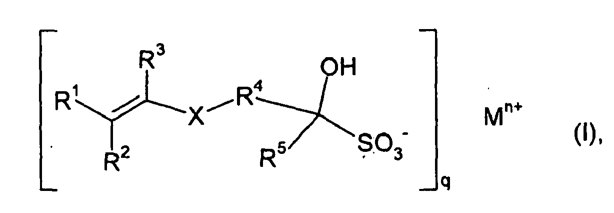 worin
R¹ und R² unabhängig voneinander Wasserstoff, Alkyl, Cycloalkyl, Aryl, Aralkyl, -COOR⁵, -COO⁻ Kat⁺ oder -CON(R⁶R⁷) bedeuten,
R⁶ und R⁷ unabhängig voneinander Wasserstoff, Alkyl oder Aryl sind,
Kat⁺ ein einwertiges Kation bedeutet, und
einer der Reste R¹ oder R² auch eine Gruppe -X-R⁴-CR⁵(OH)(SO₃⁻) bedeuten kann,
wobei X, R⁴ und R⁵ eine der unten genannten Bedeutungen annehmen,
R³ Wasserstoff, Alkyl oder Aryl bedeutet,
X ausgewählt wird aus der Gruppe direkte C-C-Bindung, -O-, -CH₂-O-, -CH₂-NR⁸-, -COO- oder -CONR⁸-,
R⁸ = Wasserstoff, Alkyl oder Aryl bedeutet,
R⁴ Alkylen, Polyoxyalkylen, Cycloalkylen oder Arylen bedeutet, und
R⁵ Wasserstoff, Alkyl, Cycloalkyl oder Aryl bedeutet.

15. Verwendung der Zusammensetzung nach einem der Ansprüche 8 bis 12 als Bindemittel in Zubereitungen zur Beschichtung von Substraten, wie z.B. in kunstharzgebundenen Putzen, Fliesenklebern, Dichtmassen und Versiegelungsmassen, Papierstreichmassen, Anstrichmitteln, als Bindemittel für Nonwovens, Glasfasern und Fiberfill, als Klebstoff zum Verkleben beliebiger Substrate, vorzugsweise poröser oder semiporöser Substrate, wie Papier, Pappe, Wellpappe, Schaumstoff, Zement, Leder, Textil oder Pressschichtstoff, als Klebstoff für den Baubereich, insbesondere als Fußboden-, Wand- oder Deckenklebstoff, oder als Möbelfolien- oder Teppichrückenkleber, als Klebstoff für die Verklebung von cellulosischen Substraten, vorzugsweise Massivholz oder von Holz abgeleiteten Materialien und Werkstoffen, inbesondere Furnieren, Sperrholz, Schichtholz, Lagenholz, Kunstharzpressholz, Verbundplatten, Holzfaserwerkstoffen oder Kunststoff-beschichteten dekorativen Holzfaserplatten, als Bindemittel für Holzfaserplatten oder Faserleder sowie als Bindemittel für Dämm-Materialien aus Papier- oder Kunststoff-Fasern, oder als Bindemittel für Putz oder Zement, als Bindemittel für textile Flächen, insbesondere für Non-Wovens, sowie im Textildruck und als Textilappretur, als Bindemittel für Glasfasern oder als Bindemittel für Keramik, als Bindemittel für Anstrichmittel, insbesondere für Dispersionsfarben, Dispersionslacke und Lasuren, oder zur Herstellung von redispergierbaren Dispersionspulvern,

## Claims

1. A copolymer derived from at least one monomer in acid or salt form containing an anion of formula (I) and one or more cations to produce electroneutrality, and from at least one other monomer copolymerizable therewith by free-radical polymerization: 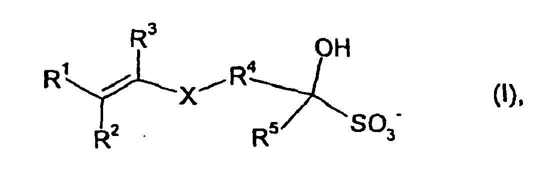 wherein
R¹ and R² are independently hydrogen, alkyl, cycloalkyl, aryl, aralkyl, -COOR⁵, -COO⁻cat⁺ or -CON(R⁶R⁷);
R⁶ and R⁷ are independently hydrogen, alkyl or aryl;
cat⁺ represents a monovalent cation; and
one of the radicals R¹ or R² may also represent a group -X-R⁴-CR⁵(OH)(SO₃⁻);
wherein cat⁺ is a monovalent cation and X, R⁴ and R⁵ have one of the meanings mentioned below;
R³ represents hydrogen, alkyl or aryl;
X is selected from the group consisting of a direct C-C bond, -O-, -CH₂-O-, -CH₂-NR⁸-, -COO- or -CONR⁸-;
R⁸ represents hydrogen, alkyl or aryl;
R⁴ represents alkylene, polyoxyalkylene, cycloalkylene or arylene; and
R⁵ represents hydrogen, alkyl, cycloalkyl or aryl.

2. The copolymer according to claim 1, **characterized in that** R¹, R² and R³ are independently hydrogen or methyl, and one of the radicals R¹ or R² may represent -COOR^{5a} or -COO-cat⁺, wherein R^{5a}, is hydrogen or C₁-C₆-alkyl.

3. The copolymer according to claim 1, **characterized in that** R⁴ represents alkylene or polyoxyalkylene, preferably unsubstituted C₁-C₆-alkylene, more preferably unsubstituted C₁-C₄-alkylene.

4. The copolymer according to any of claims 1 to 3, **characterized in that** the cations for producing electroneutrality are selected from the group consisting of mono- to polyvalent cations, preferably of hydrogen, of ammonium or of mono- to tetravalent metal ions.

5. The copolymer according to claim 1, **characterized in that** said anion of formula (I) is an anion of formula (IVa) or (IVb): 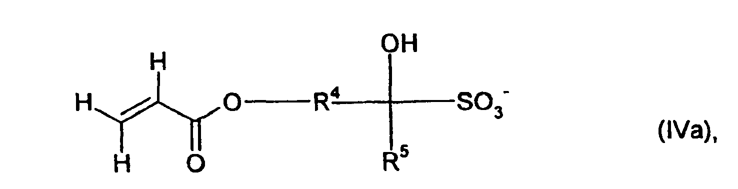 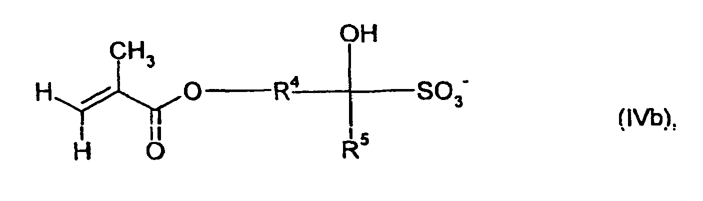 wherein R⁴ and R⁵ have the meanings as defined in claim 1.

6. The copolymer according to claim 1, **characterized in that** said other monomer copolymerizable by free-radical polymerization is selected from the group consisting of α-olefins, aliphatic hydrocarbons with two or more conjugated double bonds, vinyl esters of saturated carboxylic acids, esters of ethylenically unsaturated mono- or dicarboxylic acids, and/or alkenylaromatics.

7. The copolymer according to claim 6, **characterized in that** said copolymer contains moieties derived from ethylene, propylene, styrene, acrylate, methacrylate, vinyl esters of saturated carboxylic acids, butadiene or mixtures of two or more of these monomers, as well as up to 10% by weight of moieties derived from the monomer of formula (I), based on the total monomers.

8. A composition containing a copolymer according to claim 1 in the form of an aqueous dispersion.

9. The composition according to claim 8, **characterized by** containing at least one polyaldehyde or at least one polyhydrazine.

10. The composition according to either of claims 8 and 9, **characterized by** containing at least one at least partially masked polyaldehyde.

11. The composition according to any of claims 8 to 10, **characterized by** containing Lewis acids in the form of acidic salts of complexable metal ions, especially aluminum chloride, aluminum nitrate, zirconium oxychloride and titanium sulfate, especially the acidic salts with polyvalent complexable cations.

12. The composition according to any of claims 8 to 11, **characterized by** having a pH value of from 2.5 to 4.5.

13. A cured composition obtainable by applying and drying a composition according to any of claims 8 to 12.

14. A compound in acid or salt form containing an anion of formula (I) and one or more cations to produce electroneutrality: 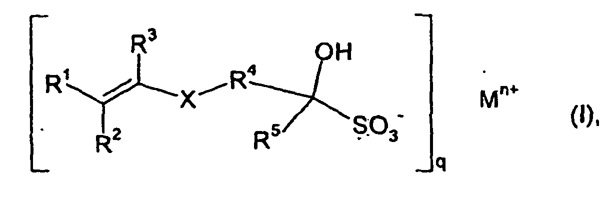 wherein
R¹ and R² are independently hydrogen, alkyl, cycloalkyl, aryl, aralkyl, -COOR⁵, -COO-cat⁺ or -CON(R⁶R⁷);
R⁶ and R⁷ are independently hydrogen, alkyl or aryl;
cat⁺ represents a monovalent cation; and
one of the radicals R¹ or R² may also represent a group -X-R⁴-CR⁵(OH)(SO₃⁻);
wherein X, R⁴ and R⁵ have one of the meanings mentioned below;
R³ represents hydrogen, alkyl or aryl;
X is selected from the group consisting of a direct C-C bond, -O-, -CH₂-O-, -CH₂-NR⁸-, -COO- or -CONR⁸-;
R⁸ represents hydrogen, alkyl or aryl;
R⁴ represents alkylene, polyoxyalkylene, cycloalkylene or arylene; and
R⁵ represents hydrogen, alkyl, cycloalkyl or aryl.

15. Use of the composition according to any of claims 8 to 12 as a binder in formulations for the coating of substrates, such as in synthetic resin bonded plasters, tile adhesives, sealing compositions, coating compositions, as a binder for non-wovens, glass fibers and fiberfill, as an adhesive for bonding any substrates, preferably porous or semiporous substrates, such as paper, cardboard, corrugated cardboard, foam, cement, leather, textile or highpressure laminate, as an adhesive for the construction field, especially as a floor, wall or ceiling adhesive, or as a furniture film adhesive or carpet backing adhesive, as an adhesive for bonding cellulosic substrates, preferably solid wood or wood-derived materials, especially veneers, plywood, glued laminated timber, laminated wood, wood plastics, laminated panels, wood fiber materials or plastic-coated decorative wood fiber boards, as binders for wood fiber boards or fiber leather, and as a binder for insulation materials of paper or plastic fibers, or as a binder for plaster or cement, as a binder for textile sheets, especially non-wovens, and in textile printing and as a textile finish, as a binder for glass fibers or as a binder for ceramics, as a binder for coating compositions, especially for emulsion paints, dispersion varnishes and glazes, or for preparing redispersible dispersion powders.

## Revendications

1. Copolymère dérivé d'au moins un monomère sous forme d'acide ou de sel contenant un anion de la formule (I) et un ou plusieurs cations pour atteindre l'électroneutralité, et au moins un autre monomère qui peut être copolymérisé avec ce dernier par polymérisation radicalaire : 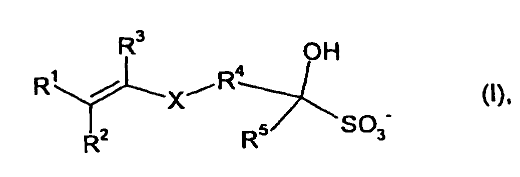 où
R¹ et R² représentent indépendamment hydrogène, alkyle, cycloalkyle, aryle, aralkyle, -COOR⁵, -COO⁻cat⁺ ou -CON(R⁶R⁷) ;
R⁶ et R⁷ représentent indépendamment hydrogène, alkyle ou aryle ;
cat⁺ représente un cation monovalent ; et
un des radicaux R¹ ou R² peut aussi représenter un groupe -X-R⁴-CR⁵(OH)(SO₃⁻) ;
où cat⁺ représente un cation monovalent et X, R⁴ et R⁵ possèdent l'une des significations mentionnées ci-dessous ;
R³ représente hydrogène, alkyle ou aryle ;
X est choisi dans le groupe consistant en une liaison C-C directe, -O-, -CH₂-O-, -CH₂-NR⁸-, -COO- ou -CONR⁸- ;
R⁸ représente hydrogène, alkyle ou aryle ;
R⁴ représente alkylène, polyoxyalkylène, cycloalkylène ou arylène ; et
R⁵ représente hydrogène, alkyle, cycloalkyle ou aryle.

2. Copolymère selon la revendication 1, **caractérisé en ce que** R¹, R² et R³ représentent indépendamment hydrogène ou méthyle, et un des radicaux R¹ ou R² peut représenter -COOR^{5a} ou -COO-cat⁺, où R^{5a} représente hydrogène ou C₁-C₆-alkyle.

3. Copolymère selon la revendication 1, **caractérisé en ce que** R⁴ représente alkylène ou polyoxyalkylène, de préférence C₁-C₆-alkylène non substitué, et de préférence encore C₁-C₄-alkylène non substitué.

4. Copolymère selon l'une quelconque des revendications 1 à 3, **caractérisé en ce que** les cations pour atteindre l'électroneutralité sont choisis dans le groupe des cations mono- à tétravalents, de préférence de l'hydrogène, de l'ammonium ou des ions métalliques mono- à tétravalents.

5. Copolymère selon la revendication 1, **caractérisé en ce que** l'anion de la formule (I) est un anion de la formule (IVa) ou (IVb) 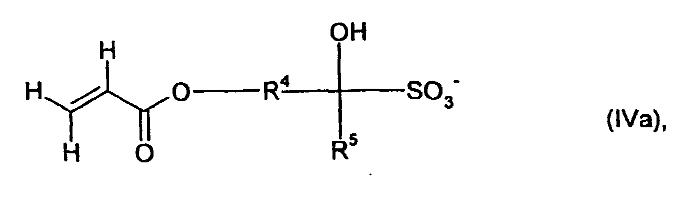 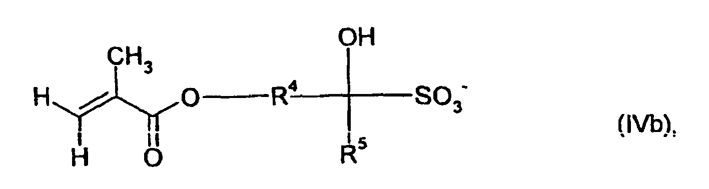 où R⁴ et R⁵ ont la signification telle que définie dans la revendication 1.

6. Copolymère selon la revendication 1, **caractérisé en ce que** l'autre monomère qui peut être copolymérisé par polymérisation radicalaire est choisi dans le groupe des α-oléfines, des hydrocarbures aliphatiques ayant deux ou plusieurs double liaisons conjuguées, des esters vinyliques d'acides carboxyliques saturés, des esters d'acides mono- ou dicarboxyliques éthyléniquement insaturés, et/ou des composés alcénylaromatiques.

7. Copolymère selon la revendication 6, **caractérisé en ce que** le copolymère contient des motifs structurels dérivés de l'éthylène, du propylène, du styrène, d'un acrylate, d'un méthacrylate, d'esters vinyliques d'acides carboxyliques saturés, du butadiène ou de mélanges de deux ou plusieurs de tels monomères, et jusqu'à 10 % en poids de motifs structurels dérivés du monomère de la formule (I), par rapport aux monomères totaux.

8. Composition contenant un copolymère selon la revendication 1 sous forme d'une dispersion aqueuse.

9. Composition selon la revendication 8, **caractérisée en ce qu'**elle contient au moins un polyaldéhyde ou au moins une polyhydrazine.

10. Composition selon l'une quelconque des revendications 8 et 9, **caractérisée en ce qu'**elle contient au moins un polyaldéhyde au moins partiellement masqué.

11. Composition selon l'une quelconque des revendications 8 à 10, **caractérisée en ce qu'**elle contient des acides de Lewis sous forme de sels acides d'ions métalliques complexables, notamment le chlorure d'aluminium, le nitrate d'aluminium, l'oxychlorure de zirconium et le sulfate de titane, et plus particulièrement les sels acides avec des cations complexables polyvalents.

12. Composition selon l'une quelconque des revendications 8 à 11, **caractérisée en ce qu'**elle a un pH compris entre 2,5 et 4,5.

13. Composition durcie que l'on peut obtenir par l'application et le séchage d'une composition selon l'une quelconque des revendications 8 à 12.

14. Composé sous forme d'acide ou de sel, contenant un anion de la formule (I) et un ou plusieurs cations pour atteindre l'électroneutralité : 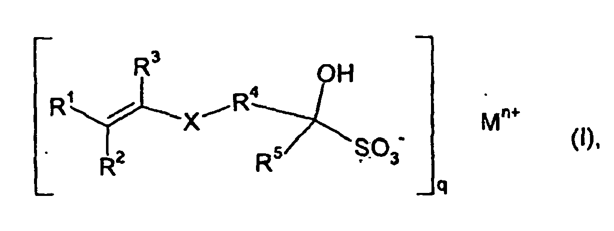 où
R¹ et R² représentent indépendamment hydrogène, alkyle, cycloalkyle, aryle, aralkyle, -COOR⁵, -COO⁻cat⁺ ou -CON(R⁶R⁷) ;
R⁶ et R⁷ représentent indépendamment hydrogène, alkyle ou aryle ;
cat⁺ représente un cation monovalent ; et
un des radicaux R¹ ou R² peut aussi représenter un groupe -X-R⁴-CR⁵(OH)(SO₃⁻) ;
où X, R⁴ et R⁵ possèdent l'une des significations mentionnées ci-dessous ; R³ représente hydrogène, alkyle ou aryle ;
X est choisi dans le groupe consistant en une liaison C-C directe, -O-, -CH₂-O-, -CH₇-N⁸-, -COO- ou -CONR⁸- ;
R⁸ représente hydrogène, alkyle ou aryle ;
R⁴ représente alkylène, polyoxyalkylène, cycloalkylène ou arylène ; et
R⁵ représente hydrogène, alkyle, cycloalkyle ou aryle.

15. Utilisation de la composition selon l'une quelconque des revendications 8 à 12 comme liant dans les préparations pour le revêtement de substrats, comme par exemple dans les enduits liés aux résines artificielles, les colles pour carreaux, les produits d'étanchéité et de scellage, les compositions de revêtement de papier, les produits de revêtement, comme liant pour les non-tissés, les fibres de verre et l'ouate synthétique, comme colle pour coller des substrats quelconques, de préférence des substrats poreux ou semi-poreux, tels que papier, carton, carton ondulé, mousse, ciment, cuir, textile ou stratifié à haute pression, comme colle pour le domaine de la construction, notamment comme colle pour revêtement de sol, adhésif mural ou colle pour dalles de plafond, ou comme adhésif pour films de meubles ou pour dossier de moquette, comme adhésif pour coller des substrats cellulosiques, de préférence du bois massif ou des matériaux dérivés de bois, notamment placages, bois contre-plaqué, bois plaqué, bois stratifié, bois comprimé à résine synthétique, panneaux composites, matériaux en fibres de bois ou panneaux de fibres de bois décoratifs enduits de plastique, comme liant pour panneaux de fibres de bois ou cuir en fibres, ainsi que comme liant pour matériaux isolants en fibres de papier ou de plastique, ou comme liant pour enduits ou ciments, comme liant pour textiles, notamment pour non-tissés, et dans l'impression textile et comme apprêtage textile, comme liant pour fibres de verre ou comme liant pour céramiques, comme liant pour produits de revêtement, notamment pour peintures émulsions synthétiques, vernis à dispersion et glacis, ou pour préparer des poudres de polymères redispersibles.
